# EUROPEAN PATENT SPECIFICATION

(11) **EP 4 593 901 B1**
(45) Date of publication and mention of the grant of the patent: **08.07.2026**
(21) Application number: 23782513.8
(22) Date of filing: 28.09.2023
(51) Int. Cl.: A61L 26/00

(54) **HAEMOSTATIC COMPOSITION**
COMPOSITION HÉMOSTATIQUE
BLUTSTILLENDE ZUSAMMENSETZUNG

(30) Priority: 30.09.2022 EP 22199052; 05.07.2023 EP 23183548
(43) Date of publication of application: 06.08.2025
(73) Proprietor: Ferrosan Medical Devices A/S, 2860 Søborg (DK)
(72) Inventor: HAMMERSHØJ, Peter Lund, 2860 Søborg (DK); BHATIA, Vikram Kjøller, 2860 Søborg (DK); BEJENARIU, Anca Gabriela, 2860 Søborg (DK); ABID, Urshan, 2860 Søborg (DK); JENNUM, Camilla Arboe, 2860 Søborg (DK); KLODZINSKA, Sylvia Natalie, 2860 Søborg (DK)
(74) Representative: Høiberg P/S
(86) International application number: PCT/EP2023/076884
(87) International publication number: WO 2024/068836

(56) References cited:
- US-A- 6 113 948
- US-A1- 2014 369 991
- US-A1- 2019 269 764
- US-A1- 2019 343 981

## Description

### Technical field

The present disclosure relates to haemostatic compositions comprising a biocompatible polymer, thrombin and fibrinogen and methods for the preparation of such compositions.

### Background

Protein-based haemostatic materials such as gelatine are commercially available in solid sponge and loose or unpacked powder form for use in surgical procedures. Mixing of the loose or unpacked powder with a fluid such as saline or a thrombin solution may form a paste or slurry that is useful as a haemostatic composition for use in cases of diffuse bleeding, particularly from uneven surfaces or hard to reach areas, depending on mixing conditions and relative ratios of the materials.

Haemostatic pastes are usually prepared at the point of use by mechanical agitation and mixing of gelatine in particulate, crosslinked form and a liquid, e.g. a thrombin solution, to provide uniformity of the composition. Mixing to form a paste usually requires extensive mixing, such as kneading or transfer between two syringes.

Surgiflo^{®} Haemostatic Matrix (Ethicon) is a commercially available kit for producing a haemostatic gelatine paste comprising thrombin, which is prepared by transferring a gelatine matrix-thrombin solution mixture back and forth between two connected syringes. Floseal^{®} Haemostatic Matrix (Baxter) is likewise a kit for producing a haemostatic gelatine paste. Once a substantially homogenous paste composition is achieved, the haemostatic pastes can be applied to a bleeding to promote haemostasis by extruding the paste from the syringe.

Thrombin is a well-known haemostatic adjuvant in haemostatic compositions, which acts as a serine protease that converts soluble fibrinogen into insoluble strands of fibrin, as well as catalysing many other coagulation-related reactions. The combination of thrombin and fibrinogen is also used in certain haemostatic products currently in clinical use, such as thrombin and fibrinogen-containing sponges, patches, glues and sealants. However, since thrombin acts on fibrinogen in the presence of water, the thrombin and fibrinogen are either provided separately or provided together in dry form to prevent premature action of thrombin on fibrinogen. For application, the thrombin and fibrinogen components can either be applied to the patient in dry form or be mixed with an aqueous medium upon application to the patient.

US 2019/269764 A1 discloses a sterile powder composition suitable for medical use comprising thrombin wherein the powder exhibits at least 500 IU's of thrombin activity per gram sterile powder composition. The composition can further comprise fibrinogen powder.

Although the currently available haemostatic products are effective in controlling mild and moderate bleedings, it would be beneficial to have haemostatic compositions that are able to control such bleedings even faster and more efficiently as well as being useful in controlling more severe bleedings. It would be even more beneficial to have haemostatic products that are efficient in controlling bleedings in minimally invasive surgical procedures where sponges, patches, glues and sealants are impractical or inefficient.

### Summary

The present disclosure provides compositions with improved haemostatic properties and methods for preparing such haemostatic compositions. The improved haemostatic properties are due to an optimised combination of components leading to improved adhesive properties. Such compositions are highly valuable in the operating room, where bleeding must be controlled in a fast and efficient manner.

Surprisingly, the present inventors have shown that the haemostatic paste compositions disclosed herein comprising a biocompatible polymer, thrombin and fibrinogen remain flowable after reconstitution for more than 1.5 hours. Accordingly, the paste compositions of the present disclosure can be prepared in advance and used for an extended period of time, which is highly advantageous in the operating room and in advanced surgical procedures.

Thus, in one aspect, the present disclosure provides a haemostatic composition comprising:
a) a compatible polymer in particulate form selected from gelatine, collagen, chitin, chitosan, alginate, cellulose, oxidised cellulose, carboxymethylcellulose, polyglycolic acid, polyacetic acid and combinations thereof,
b) fibrinogen in an amount of 1 to 150 mg per gram of biocompatible polymer, and
c) thrombin in an amount of 100 to 5000 IU per gram of biocompatible polymer.

In a second aspect, the present disclosure relates to a method for preparing a haemostatic composition comprising the steps of:
a) providing the dry haemostatic composition as described herein, and
b) adding an amount of an aqueous medium to the dry haemostatic composition of a).

The amount of aqueous medium added is usually an amount sufficient to achieve a haemostatic composition in the form of a paste.

In another aspect, the present disclosure provides a haemostatic composition comprising:
a) a biocompatible polymer in particulate form in an amount of 7 to 34% by weight, wherein said biocompatible polymer is selected from gelatine, collagen, chitin, chitosan, alginate, cellulose, oxidised cellulose, carboxymethylcellulose, polyglycolic acid, polyacetic acid and combinations thereof,
b) fibrinogen in an amount of 0.008 to 5% by weight,
c) thrombin in an amount of 7 to 1700 IU per gram, and
d) an aqueous medium.

The haemostatic composition is usually provided in the form of a paste, i.e. the aqueous medium is present in an amount sufficient to provide a composition in paste form.

In yet another aspect, the present disclosure relates to the haemostatic composition described herein for use in promoting haemostasis and/or wound, bone, tendon and/or tissue healing in an individual in need thereof.

In another aspect, the present disclosure relates to a container comprising the haemostatic composition described herein.

In a final aspect, the present disclosure provides a kit comprising;
a) a first container comprising a haemostatic composition as described herein,
b) a second container comprising an aqueous medium; and
c) optionally an outer package,
wherein the two containers are interconnectable.

### Definitions

The term "about" as used herein to refer to an amount or percentage is to be interpreted as a variation of ± 10% with respect the value of the amount or percentage it refers to, such as ± 5%.

A "bioactive agent" is any agent, drug, compound, composition of matter or mixture which provides some pharmacologic, often beneficial, effect that can be demonstrated in vivo or in vitro. An agent is thus considered bioactive if it has interaction with or effect on a cell tissue in the human or animal body. As used herein, this term further includes any physiologically or pharmacologically active substance that produces a localized or systemic effect in an individual. Bioactive agents may be a protein, such as an enzyme. Further examples of bioactive agents include, but are not limited to, agents comprising or consisting of an oligosaccharide, a polysaccharide, an optionally glycosylated peptide, an optionally glycosylated polypeptide, an oligonucleotide, a polynucleotide, a lipid, a fatty acid, a fatty acid ester and secondary metabolites. It may be used either prophylactically, therapeutically, in connection with treatment of an individual, such as a human or any other animal. The term "bioactive agent" as used herein does not encompass cells, such as eukaryotic or prokaryotic cells.

"Biocompatible" refers to a material's ability to perform its intended function without eliciting any substantial undesirable local or systemic effects in the host.

"Biologically absorbable" or "resorbable" are terms which in the present context are used to describe that the materials of which the said powder are made can be degraded in the body to smaller molecules having a size which allows them to be transported into the blood stream. By said degradation and absorption the said powder materials will gradually be removed from the site of application. For example, gelatine can be degraded by proteolytic tissue enzymes to absorbable smaller molecules, whereby the gelatine, when applied in tissues, typically is absorbed within about 4-6 weeks and when applied on bleeding surfaces and mucous membranes typically within 3-5 days.

"Haemostasis" is a process which causes bleeding to diminish or stop. Haemostasis occurs when blood is present outside of the body or blood vessels and is the instinctive response for the body to stop bleeding and loss of blood. During haemostasis three steps occur in a rapid sequence. Vascular spasm is the first response as the blood vessels constrict to allow less blood to be lost. In the second step, platelet plug formation, platelets stick together to form a temporary seal to cover the break in the vessel wall. The third and last step is called coagulation or blood clotting. Coagulation reinforces the platelet plug with fibrin threads that act as a "molecular glue". Accordingly, a haemostatic compound is capable of stimulating haemostasis.

"International Unit (IU)". In pharmacology, the International Unit is a unit of measurement for the amount of a substance, based on biological activity or effect. It is abbreviated as IU, UI, or as IE. It is used to quantify vitamins, hormones, some medications, vaccines, blood products, and similar biologically active substances.

A "paste" according to the present disclosure has a malleable, putty-like consistency, such as toothpaste. A paste is a thick fluid mixture of pulverized solid/solid in powder form with a liquid. A paste is a substance that behaves as a solid until a sufficiently large load or stress is applied, at which point it flows like a fluid, i.e. a paste is flowable. Flowables conform efficiently to irregular surfaces upon application. Pastes typically consist of a suspension of granular material in a background fluid. The individual grains are jammed together like sand on a beach, forming a disordered, glassy or amorphous structure, and giving pastes their solid-like character. It is this "jamming together" that gives pastes some of their most unusual properties; this causes a paste to demonstrate properties of fragile matter. A paste is not a gel/jelly. A "slurry" is a fluid mixture of a powdered/pulverized solid with a liquid, such as water. Slurries behave in some ways like thick fluids, flowing under gravity and being capable of being pumped if not too thick. A slurry may functionally be regarded as a thin, watery paste, but a slurry generally contains more water than a paste. Substantially water-insoluble powder particles, such as cross-linked gelatine particles, will form a paste or slurry upon mixing with an aqueous medium.

"Percentage". If nothing else is indicated, the percentage is percentage by weight: % w/w or wt%. Ratios are indicated as weight by weight (w/w).

### Detailed description

The present disclosure provides compositions with improved haemostatic properties and methods to prepare said haemostatic compositions.

Thus, in one aspect, the present disclosure provides a haemostatic composition comprising:
a) a biocompatible polymer in particulate form selected from gelatine, collagen, chitin, chitosan, alginate, cellulose, oxidised cellulose, carboxymethylcellulose, polyglycolic acid, polyacetic acid and combinations thereof,
b) fibrinogen in an amount of 1 to 150 mg per gram of biocompatible polymer, and
c) thrombin in an amount of 100 to 5000 IU per gram of biocompatible polymer.

In a second aspect, the present disclosure relates to a method for preparing a haemostatic composition comprising the steps of:
a) providing the dry haemostatic composition as described herein, and
b) adding an amount of an aqueous medium to the dry haemostatic composition of a).

The advantages of the haemostatic compositions disclosed herein are numerous and include:
- Improved haemostatic effect, e.g. more severe bleedings can be stopped in less time.
- Less time spent preparing the haemostatic composition, e.g. bleeding can be stopped faster.
- Decreased risk of compromising the sterility of the haemostatic composition during preparation due to less handling steps.
- Decreased risk of making mistakes during preparation due to the simplified preparation of the paste.
- Reliable and consistent reconstitution within a short time period.
- Superior consistency and adhesive properties decreasing the need for compression.
- Superior for Minimally Invasive Surgery (MIS) including robotic surgery.
- Application in sprayable patches possible.
- Avoids the time-consuming and error-prone dilution steps of standard haemostatic composition preparations.
- Minimises Operation Room costs since preparation of the currently described product is so simple and fast that there is no reason to pre-prepare haemostatic flowables before surgery which need to be discarded.

### Dry haemostatic composition

In one embodiment, the present disclosure relates to a haemostatic composition comprising:
a) a biocompatible polymer in particulate form selected from gelatine, collagen, chitin, chitosan, alginate, cellulose, oxidised cellulose, carboxymethylcellulose, polyglycolic acid, polyacetic acid and combinations thereof,
b) fibrinogen in an amount of 1 to 150 mg per gram of biocompatible polymer, and
c) thrombin in an amount of 100 to 5000 IU per gram of biocompatible polymer.

The haemostatic composition is usually provided in substantially dry, storage stable form. In one embodiment, the dry composition is storage stable at room temperature for at least 12 months, preferably at least 24 months.

In one embodiment, the haemostatic composition comprises the one or more biocompatible polymer in particulate form in an amount of at least 80% by weight of the composition, such as at least 81% by weight, such as at least 83% by weight, such at least 85% by weight, such as at least 87% by weight, such as at least 90% by weight, such as at least 91% by weight, such as at least 95% by weight of the composition.

In one embodiment, the haemostatic compositions comprises the one or more biocompatible polymer in particulate form in an amount between 80% to 99% by weight of the composition, such as between 81% to 99%, such as between 82% to 99%, such as between 83% to 99%, such as between 84% to 99%, such as between 85% to 99%, such as between 86% to 99%, such as between 87% to 99%, such as between 88% to 99%, such as between 89% to 99%, such as between 90% to 99% by weight of the composition.

In one embodiment, the haemostatic compositions comprises the one or more biocompatible polymer in particulate form in an amount between 85% to 99% by weight of the composition, such as between 85% to 98%, such as between 85% to 97%, such between as 85% to 96%, such as between 85% to 96%, such as between 85 % to 95 % by weight of the composition.

For example, in one embodiment, the haemostatic composition comprises the one or more biocompatible polymers in particulate form in an amount of 83% to 97% by weight of the composition, such as 83%, 84%, 85%, 86%, 87%, 88%, 89%, 90%, 91%, 92%, 93%, 94%, 95%, 96% or 97% by weight of the composition.

In one embodiment, the haemostatic composition comprises the one or more biocompatible polymers in particulate form in an amount of 85 % to 97% by weight of the composition. In one embodiment, the haemostatic composition comprises the one or more biocompatible polymers in particulate form in an amount of 89 % to 97% by weight of the composition. In one embodiment, the haemostatic composition comprises the one or more biocompatible polymers in particulate form in an amount of 90% to 97% by weight of the composition. In one embodiment, the haemostatic composition comprises the one or more biocompatible polymers in particulate form in an amount of 92% to 97% by weight of the composition. In one embodiment, the haemostatic composition comprises the one or more biocompatible polymers in particulate form in an amount of 92 %, 93 %, 94 %, 95 %, 96 % or 97% by weight of the composition.

In one embodiment, present disclosure relates to a haemostatic composition comprising:
a) a biocompatible polymer in particulate form selected from gelatine, collagen, chitin, chitosan, alginate, cellulose, oxidised cellulose, carboxymethylcellulose, polyglycolic acid, polyacetic acid and combinations thereof,
b) fibrinogen in an amount of 1 to 150 mg per gram of biocompatible polymer, and
c) thrombin in an amount of 100 to 5000 IU per gram of biocompatible polymer;
wherein the one or more biocompatible polymers in particulate form is present in an amount of at least 80% by weight of the composition, such as at least 81% by weight, such as at least 83% by weight, such at least 85% by weight, such as at least 87% by weight, such as at least 90% by weight, such as at least 91% by weight, such as at least 95% by weight of the composition.

In one embodiment, present disclosure relates to a haemostatic composition comprising:
a) a biocompatible polymer in particulate form selected from gelatine, collagen, chitin, chitosan, alginate, cellulose, oxidised cellulose, carboxymethylcellulose, polyglycolic acid, polyacetic acid and combinations thereof,
b) fibrinogen in an amount of 1 to 150 mg per gram of biocompatible polymer, and
c) thrombin in an amount of 100 to 5000 IU per gram of biocompatible polymer;
wherein the one or more biocompatible polymers in particulate form is present in an amount of 85 % to 97 % by weight of the composition, such as 86 %, 87 %, 88 %, 89 %, 90 %, 91 %, 92 %, 93 %, 94 %, 95 %, 96 % or 97% by weight of the composition.

In one embodiment, present disclosure relates to a haemostatic composition comprising:
a) a biocompatible polymer in particulate form selected from gelatine, collagen, chitin, chitosan, alginate, cellulose, oxidised cellulose, carboxymethylcellulose, polyglycolic acid, polyacetic acid and combinations thereof,
b) fibrinogen in an amount of 1 to 150 mg per gram of biocompatible polymer, and
c) thrombin in an amount of 100 to 5000 IU per gram of biocompatible polymer;
wherein, the one or more biocompatible polymers in particulate form is present in an amount of 90 % to 97 % by weight of the composition.

In one embodiment the haemostatic composition contains less than 10% water by weight, preferably less than 5% water by weight, preferably less than 1% water by weight.

### Biocompatible polymer

The biocompatible polymer of the present disclosure may be a biologic or a non-biologic polymer. Suitable biologic polymers include proteins, such as gelatine, collagen, albumin, hemoglobin, casein, fibrinogen, fibrin, fibronectin, elastin, keratin, and laminin; or derivatives or combinations thereof. Particularly preferred is the use of gelatine or collagen, more preferably gelatine. Other suitable biologic polymers include polysaccharides, such as glycosaminoglycans, starch derivatives, xylan, cellulose derivatives, hemicellulose derivatives, agarose, alginate, and chitosan; or derivatives or combinations thereof. Suitable non-biologic polymers will be selected to be degradable by either of two mechanisms, i.e. (1) break down of the polymeric backbone or (2) degradation of side chains which result in aqueous solubility. Exemplary nonbiologic polymers include synthetics, such as polyacrylates, polymethacrylates, polyacrylamides, polyvinyl resins, polylactide- glycolides, polycaprolactones, and polyoxyethylenes; or derivatives or combinations thereof. Also combinations of different kinds of polymers are possible.

The biocompatible polymer in particulate form selected from the group consisting of: gelatine, collagen, chitin, chitosan, alginate, cellulose, oxidised cellulose, carboxymethylcellulose, polyglycolic acid, polyacetic acid and combinations thereof.

In one embodiment, the biocompatible polymer comprises or consists of powder particles, which are substantially insoluble in an aqueous medium.

In one embodiment, the biocompatible polymer is biologically absorbable. Examples of suitable biologically absorbable materials include gelatine, collagen, chitin, chitosan, alginate, cellulose, oxidised cellulose, polyglycolic acid, polyacetic acid and combinations thereof. It will be understood that various forms thereof, such as linear or cross-linked forms, salts, esters and the like are also contemplated for the present disclosure. In a preferred embodiment of the invention, the biologically absorbable material comprises or consists of gelatine. Gelatine is preferred since gelatine is highly biologically absorbable. Furthermore, gelatine is highly biocompatible, meaning that it is non-toxic to an animal, such as a human being, when/if entering the blood stream or being in long-term contact with human tissues.

The gelatine typically originates from a porcine source, but may originate from other animal sources, such as from bovine or fish sources. The gelatine may also be synthetically made, i.e. made by recombinant means.

In a preferred embodiment, the biocompatible polymer is cross-linked. Cross-linking usually renders the polymer substantially insoluble in an aqueous medium. In one embodiment, the biocompatible polymer consists of powder particles which are substantially insoluble in an aqueous medium. Any suitable cross-linking methods known to a person of skill may be used including both chemical and physical cross-linking methods.

In one embodiment of the present disclosure the polymer has been cross-linked by physical means, such as by dry heat. The dry heat treatment is usually performed at temperatures between 100°C and 250°C, such as about 110°C to about 200°C. In particular the temperature may be in the range of 110-160°C, e.g. in the range of 110-140°C, or in the range of 120-180°C, or in the range of 130-170°C, or in the range of 130-160°C, or in the range of 120-150°C. The period of time for cross-linking may be optimised by a skilled person and is normally a period between about 10 minutes to about 12 hours, such as about 1 hour to about 10 hours, for example between about 2 hours to about 10 hours, such as between about 4 hours to about 8 hours, for example between about 5 hours to about 7 hours, such as about 6 hours.

In another embodiment, the polymer has been cross-linked by chemical means, i.e. by exposure to a chemical cross-linking agent. Examples of suitable chemical cross-linking agents include but are not limited to aldehydes, in particular glutaraldehyde and formaldehyde, acyl azide, carbodiimides, hexamethylene diisocyanate, polyether oxide, 1,4-butanedioldiglycidyl ether, tannic acid, aldose sugars, e.g. D-fructose, genipin and dye-mediated photo-oxidation. Specific compounds include but are not limited to 1-(3-dimethylaminopropyl)-3-ethylcarbodiimide hydrochloride (EDC) and dithiobis(propanoic dihydrazide) (DTP).

In one embodiment, the biocompatible polymer particles according to the present disclosure are obtained from cross-linked sponges of e.g. gelatine or collagen, in particular cross-linked sponges of gelatine (such as the commercially available Spongostan^{®} sponges and Surgifoam^{®} sponges). The cross-linked sponges are micronized by methods known in the art to obtain a cross-linked biocompatible polymer in powder form, such as by rotary bed, extrusion, granulation and treatment in an intensive mixer, or milling (e.g. by using a hammer mill or a centrifugal mill).

Spongostan^{®}/Surgifoam^{®} available from Ethicon is a gelatine based cross-linked absorbable haemostatic sponge. It absorbs > 35 g of blood/g and within 4-6 weeks it is completely absorbed in the human body.

In one embodiment, the biocompatible polymer in particulate form comprises or consists of cross-linked gelatine particles.

In one embodiment, the cross-linked gelatine particles are obtained from a micronized porous gelatine sponge or dried hydrogel. In one embodiment, the porous gelatine sponge or dried hydrogel has been cross-linked by dry heat treatment.

Porous gelatine sponges may be prepared by mixing an amount of soluble gelatine with an aqueous medium in order to create a foam comprising a discontinuous gas phase, drying said foam and crosslinking the dried foam by exposure to dry heat. The obtained cross-linked sponge can be micronized by methods known in the art. The gelatine foam usually has a gelatine concentration from about 1% to 70% by weight, usually from 3% to 20% by weight. Drying is usually performed at about 20°C to about 40°C for about 5 to 20 hours. The dried foam is usually cross-linked by exposure to a temperature of about 110°C to about 200°C for about 15 minutes to about 8 hours, such as at about 150°C to about 170°C for about 5 to 7 hours. The period of time for cross-linking may be optimised by a skilled person and is normally a period between about 10 minutes to about 12 hours, such as about 1 hour to about 10 hours, for example between about 2 hours to about 10 hours, such as between about 4 hours to about 8 hours, for example between about 5 hours to about 7 hours, such as about 6 hours.

Drying of the foam or hydrogel may also be achieved by freeze-drying by methods known to a person skilled in the art.

In one embodiment, the cross-linked gelatine particles are obtained by micronizing a crosslinked porous gelatine sponge or a substantially non-porous crosslinked dried hydrogel. The gelatine may be cross-linked, for example by exposure to either glutaraldehyde (e.g. 0.01% to 0.05% w/w, overnight at 0°C to 15°C in aqueous buffer), sodium periodate (e.g. 0.05 M, held at 0°C to 15°C for 48 hours) or 1-ethyl-3-(3-dimethylaminopropyl) carbodiimide (EDC) (e.g. 0.5% to 1.5% w/w, overnight at room temperature), or by exposure to about 0.3 to 3 megarads of gamma or electron beam radiation. When cross-linking with glutaraldehyde, the cross-links are formed via Schiff bases which may be stabilized by subsequent reduction, e.g. by treatment with sodium borohydride.

Particles may be obtained from the dried product by methods known to a person skilled in the art. In one embodiment the gelatine particles are obtained by micronization of the dry product, such as by granulation and treatment in an intensive mixer, milling, e.g. by using a hammer mill, ball milling, or a centrifugal mill. In another embodiment, the particles are obtained by grinding of the dry product to an appropriate size. This can e.g. be done by mortar and pestle, crushing and any other available physical process.

In one embodiment, the particles are obtained by hammer milling of a sponge or dried hydrogel. Preferably, the hammer mill has a built-in sieve resulting in a desired particle size distribution.

In one embodiment of the present invention, the gelatine particles have a diameter between about 1 µm and 1000 µm, such as between about 10 µm and 800 µm, for example between about 50 µm and 600 µm, such as between about 100 µm and 500 µm, for example between about 200 µm and 500 µm, such as about 450 µm.

The particles are in one embodiment less than approximately 1000 microns in size, i.e. so that they are able to pass through a 1x1 mm sieve.

Generally, at least 90% of the powder particles have a size of between 1 µm and 1200 µm.

In another embodiment, the average particle size of the dry particles is between 1 µm and 1000 µm, such as between about 10 µm and 800 µm, for example between about 50 µm and 600 µm, such as between about 100 µm and 500 µm, for example between about 200 µm and 500 µm, such as about 450 µm.

The average particle size of the dry particles can e.g. be measured by laser diffraction.

In one embodiment, the biocompatible polymer in particulate form is present in an amount from about 0.05 to 20 g, such as from about 0.2 to 10 g, such as from about 0.5 to 2g, such as about 1 g.

In one embodiment, the biocompatible polymer in particulate form is present in an amount of about 1 g.

The biocompatible polymers in particulate form used in the present disclosure are usually provided in sterile form.

### Fibrinogen

Fibrinogen or factor I is a glycoprotein complex that circulates in the blood of vertebrates. During tissue and vascular injury, it is converted enzymatically by thrombin to fibrin and is involved in the blood clot formation.

In one embodiment, the fibrinogen is human fibrinogen.

In one embodiment, the fibrinogen is recombinant human fibrinogen.

In other embodiments, the origin of the fibrinogen is from a mammal other than human, such as bovine fibrinogen.

In one embodiment, the fibrinogen is a dry fibrinogen composition. For example, the fibrinogen may be in the form of particles or a powder. The dry fibrinogen composition may be may be prepared by any methods known to the skilled person and is usually provided in sterile form. Thus, in one embodiment the dry fibrinogen composition is sterile.

The fibrinogen may be also coated onto the biocompatible polymer in particulate form. The coating of the fibrinogen onto the biocompatible polymer may be obtained by any methods known in the art, for example by spraying techniques, which can be performed in any spraying apparatus. For example, one well-known method for coating particles by spraying is a fluid bed process. Thus in one embodiment, the fibrinogen is coated onto the biocompatible polymer particles so as to obtain a layer of fibrinogen on the biocompatible polymer particle. In one embodiment, the fibrinogen is sprayed onto the biocompatible polymer particles, e.g by fluid bed process techniques.

In one embodiment, the haemostatic composition comprises fibrinogen in an amount of about 1 to about 150 mg per gram of biocompatible polymer, such as from about 5 to about 150 mg of fibrinogen per gram of the biocompatible polymer, such as from about 10 to about 150 mg, such as from about 15 to about 150 mg, such as from about 20 to about 150 mg, such as from about 25 to about 150 mg, such as from about 30 to about 150 mg, such as from about 30 to about 125 mg, such as from about 30 to about 100 mg of fibrinogen per gram of the biocompatible polymer.

In one embodiment, the haemostatic composition comprises fibrinogen in an amount from about 20 to about 80 mg of fibrinogen per gram of the biocompatible polymer, such as about 30 mg, such as about 35 mg, such as about 40 mg, such as about 45 mg, such as about 50 mg, such as about 55 mg, such as about 60 mg, such as about 70 mg, such as about 75 mg of fibrinogen per gram of the biocompatible polymer. In one embodiment, the composition comprises about 35 mg of fibrinogen per gram of the biocompatible polymer. In one embodiment, the composition comprises about 70 mg of fibrinogen per gram of the biocompatible polymer.

In one embodiment, the haemostatic composition comprises fibrinogen in an amount from about 20 mg to about 100 mg of fibrinogen per gram of the biocompatible polymer, such as from about 25 mg to about 100mg, such as from about 30 mg to about 100mg, such as from 30 mg to about 95 mg, such as from 30 mg to about 90 mg, such as from 30 mg to about 85 mg of fibrinogen per gram of the biocompatible polymer.

In one embodiment, the haemostatic composition comprises no more than 100 mg of fibrinogen per gram of the biocompatible polymer. In one embodiment, the composition comprises more than 10 mg of fibrinogen per gram of the biocompatible polymer.

In one embodiment, the haemostatic composition comprises from about 20 to about 100 mg of fibrinogen per gram of the biocompatible polymer, such as from about 20 to about 30 mg, such as from about 30 mg to about 40 mg, such as from about 40 mg to about 50 mg, such as from about 50 mg to about 60 mg, such as from about 60 mg to about 70 mg, such as from 70 mg to about 80 mg, such as from 80 mg to about 90 mg, such as from 90 mg to about 100 mg of fibrinogen per gram of the biocompatible polymer.

In one embodiment, the haemostatic composition, when in the dry form, i.e. prior to mixing with an aqueous medium, comprises fibrinogen in an amount from 1 to 15% by weight, such as 1 to 10% by weight, such as such as from 1 to 2%, such as from 2 to 3%, such as from 3 to 4%, such as from 4 to 5%, such as from 5 to 6%, such as from 6 to 7%, such as from 7 to 8%, such as from 8 to 9%, such as from 9 to 10% by weight.

In one embodiment, the haemostatic composition, prior to mixing with an aqueous medium, comprises fibrinogen in an amount from 2 to 15% by weight, such as from 2.5% to 15% by weight, such as from 3% to 15% by weight, such as about 3% by weight, such as about 3.5% by weight, such as about 4% by weight, such as about 4.5% by weight, such as about 5% by weight, such as about 5.5% by weight, such as about 6% by weight, such as about 6.5% by weight, such as about 7% by weight, such as about 7.5% by weight, such as about 8% by weight, such as about 8.5% by weight, such as about 9% by weight, such as about 9.5% by weight, such as about 10% by weight.

### Thrombin

Thrombin is a "trypsin-like" serine protease protein that in humans is encoded by the F2 gene. Prothrombin (coagulation factor II) is proteolytically cleaved to form thrombin in the coagulation cascade, which ultimately results in the stemming of blood loss. Thrombin in turn acts as a serine protease that converts soluble fibrinogen into insoluble strands of fibrin, as well as catalysing many other coagulation-related reactions. In the blood coagulation pathway, thrombin acts to convert factor XI to Xla, VII to VIIla, V to Va, and fibrinogen to fibrin.

In one embodiment, the thrombin is human thrombin.

In one embodiment, the thrombin is recombinant human thrombin.

In other embodiments, the origin of the thrombin is from a mammal other than human, such as bovine thrombin.

In one embodiment, the thrombin is in the form of prothrombin.

In one embodiment, the thrombin is a dry thrombin composition. For example, the thrombin may be in the form of particles or a powder.The dry thrombin composition may be may be prepared by any methods known to the skilled person and is usually provided in sterile form. Thus, in one embodiment the dry thrombin composition is sterile.

The thrombin may be also coated onto the biocompatible polymer in particulate form. The coating of the thrombin onto the biocompatible polymer may be obtained by any methods known in the art, for example by spraying techniques, which can be performed in any spraying apparatus. For example, a well-known method for coating particles by spraying is a fluid bed process. Thus in one embodiment, the thrombin is coated onto the biocompatible polymer particles so as to obtain a layer of thrombin on the biocompatible polymer particle. In one embodiment, the thrombin is sprayed onto the biocompatible polymer particles.

In some embodiments, the biocompatible polymer in particulate form is coated with both thrombin and fibrinogen using known methods of coating. A person of skill in the art will be aware on how to perform such coatings in a spraying apparatus, such as in a fluid bed process. For example, a first coating of fibrinogen or thrombin can be applied onto the biocopmpatible polymer particles, so as to obtain a first layer on the biocompatible polymer particles. Thereafter, a second coating of thrombin or fibrinogen may be applied, so as to obtain a second layer on the first layer.

It is also possible to combine both thrombin and fibrinogen in the same layer. For example, by simultaneously coating both thrombin and fibrinogen onto the biocompatible polymers particles so as to obtain a layer combining fibrinogen and thrombin. For example, thrombin and fibrinogen may each be sprayed simultaneously from separate spraying sources, or sprayed from the same spraying source.

In some embodiments, the medium used to spray thrombin and/or fibrinogen is a nonaqueous medium so as to prevent the catalytic reaction of thrombin on fibrinogen.

In one embodiment, a mixture of particles coated with thrombin and fibrinogen, respectively, is obtained by coating one subset of polymer particles with thrombin and another subset of polymer particles with fibrinogen and then combining the two coated subsets in the right ratio as as to obtain an optimal polymer:fibrinogen:thrombin ratio.

In one embodiment, the dry thrombin composition is prepared by spray-drying or freeze-drying.

In one embodiment, the dry thrombin composition is prepared by freeze-drying.

In one embodiment, the dry thrombin composition comprises less than 2% water, such as less than 1% water.

In one embodiment, the haemostatic composition comprises thrombin in an amount from 400 to 4000 IU of thrombin per gram of the biocompatible polymer, such as from 400 to 600 IU, such as from 600 to 800 IU, such as from 800 to 1000 IU, such as from 1000 to 1200 IU, such as from 1200 to 1400 IU, such as from 1400 to 1600 IU, such as from 1600 to 1800 IU, such as from 1800 to 2000 IU, such as from 2000 to 2200 IU, such as from 2200 to 2400 IU, such as from 2400 to 2600 IU, such as from 2600 to 2800 IU, such as from 2800 to 3000 IU, such as from 3000 to 3200 IU, such as from 3200 to 3400 IU, such as from 3400 to 3600 IU, such as from 3600 to 3800 IU, such as from 3800 to 4000 IU, such as from 4000 to 4200 IU of thrombin per gram of the biocompatible polymer.

In one embodiment, the haemostatic composition comprises thrombin in an amount from about 500 IU to 2500 IU of thrombin per gram of biocompatible polymer, such as from about 600 IU to 2500 IU, such as from about 700 IU to 2500 IU, such as from about 800 IU to 2500 IU, such as from about 900 IU to 2500 IU, such as from about 1000 IU to 2500 IU, such as from about 1100 IU to 2500 IU, such as from about 1200 IU to 2500 IU, such as from about 1300 IU to 2500 IU, such as from about 1400 IU to 2500 IU, such as from about 1500 IU to 2500 IU, such as from about 1600 IU to 2500 IU, such as from about 1700 IU to 2500 IU, such as from about 1800 IU to 2500 IU, such as from about 1900 IU to 2500 IU of thrombin per gram of biocompatible polymer, such as about 500 IU, such as about 1000 IU, such as about 1500 IU, such as about 2000 IU, such as about 2500 IU of thrombin per gram of biocompatible polymer. In one embodiment, the composition comprises about 2000 IU of thrombin per gram of biocompatible polymer.

In one embodiment, the haemostatic composition comprises a ratio of thrombin to fibrinogen from 0.5 IU/mg to 5000 IU/mg, such as from 1 IU/mg to 2000 IU/mg, such as 2 IU/mg to 1000 IU/mg, such as from 2 IU/mg to 300 IU/mg, such as from 2 IU/mg to 250 IU/mg, such as from 2 IU/mg to 200 IU/mg, such as from 2 IU/mg to 150 IU/mg.

In one embodiment the haemostatic composition comprises 30 to 40 mg fibrinogen and 500 to 1000 IU of thrombin per gram of biocompatible polymer. In one embodiment the haemostatic composition comprises 30 to 40 mg fibrinogen and 1000 to 1500 IU of thrombin per gram of biocompatible polymer. In one embodiment the haemostatic composition comprises 30 to 40 mg fibrinogen and 1500 to 2000 IU of thrombin per gram of biocompatible polymer. In one embodiment the haemostatic composition comprises 30 to 40 mg fibrinogen and 2000 to 2500 IU of thrombin per gram of biocompatible polymer. In one embodiment the haemostatic composition comprises 40 to 50 mg fibrinogen and 500 to 1000 IU of thrombin per gram of biocompatible polymer. In one embodiment the haemostatic composition comprises 40 to 50 mg fibrinogen and 1000 to 1500 IU of thrombin per gram of biocompatible polymer. In one embodiment the haemostatic composition comprises 40 to 50 mg fibrinogen and 1500 to 2000 IU of thrombin per gram of biocompatible polymer. In one embodiment the haemostatic composition comprises 40 to 50 mg fibrinogen and 2000 to 2500 IU of thrombin per gram of biocompatible polymer. In one embodiment the haemostatic composition comprises 50 to 60 mg fibrinogen and 500 to 1000 IU of thrombin per gram of biocompatible polymer. In one embodiment the haemostatic composition comprises 50 to 60 mg fibrinogen and 1000 to 1500 IU of thrombin per gram of biocompatible polymer. In one embodiment the haemostatic composition comprises 50 to 60 mg fibrinogen and 1500 to 2000 IU of thrombin per gram of biocompatible polymer. In one embodiment the haemostatic composition comprises 50 to 60 mg fibrinogen and 2000 to 2500 IU of thrombin per gram of biocompatible polymer. In one embodiment the haemostatic composition comprises 60 to 70 mg fibrinogen and 500 to 1000 IU of thrombin per gram of biocompatible polymer. In one embodiment the haemostatic composition comprises 60 to 70 mg fibrinogen and 1000 to 1500 IU of thrombin per gram of biocompatible polymer. In one embodiment the haemostatic composition comprises 60 to 70 mg fibrinogen and 1500 to 2000 IU of thrombin per gram of biocompatible polymer. In one embodiment the haemostatic composition comprises 60 to 70 mg fibrinogen and 2000 to 2500 IU of thrombin per gram of biocompatible polymer.

In one embodiment the haemostatic composition comprises 35 mg fibrinogen and 500 to 1000 IU of thrombin per gram of biocompatible polymer. In one embodiment the haemostatic composition comprises 35 mg fibrinogen and 1000 to 1500 IU of thrombin per gram of biocompatible polymer. In one embodiment the haemostatic composition comprises 35 mg fibrinogen and 1500 to 2000 IU of thrombin per gram of biocompatible polymer. In one embodiment the haemostatic composition comprises 35 mg fibrinogen and 2000 to 2500 IU of thrombin per gram of biocompatible polymer. In one embodiment the haemostatic composition comprises 50 mg fibrinogen and 500 to 1000 IU of thrombin per gram of biocompatible polymer. In one embodiment the haemostatic composition comprises 50 mg fibrinogen and 1000 to 1500 IU of thrombin per gram of biocompatible polymer. In one embodiment the haemostatic composition comprises 50 mg fibrinogen and 1500 to 2000 IU of thrombin per gram of biocompatible polymer. In one embodiment the haemostatic composition comprises 50 mg fibrinogen and 2000 to 2500 IU of thrombin per gram of biocompatible polymer. In one embodiment the haemostatic composition comprises 70 mg fibrinogen and 500 to 1000 IU of thrombin per gram of biocompatible polymer. In one embodiment the haemostatic composition comprises 70 mg fibrinogen and 1000 to 1500 IU of thrombin per gram of biocompatible polymer. In one embodiment the haemostatic composition comprises 70 mg fibrinogen and 1500 to 2000 IU of thrombin per gram of biocompatible polymer. In one embodiment the haemostatic composition comprises 70 mg fibrinogen and 2000 to 2500 IU of thrombin per gram of biocompatible polymer.

In a particular embodiment the haemostatic composition comprises:
a) about 1g of a biocompatible polymer in particulate form, such as gelatine,
b) about 35 mg of fibrinogen,
c) about 2000 IU of thrombin.

In one embodiment, the haemostatic composition contains less than 10% water by weight, preferably less than 5% water by weight, preferably less than 1% water by weight.

### Hydrophilic compounds

In one embodiment, the haemostatic composition further comprises one or more hydrophilic compounds. Hydrophilic compounds usually contain polar or charged functional groups, rendering them soluble in water. Inclusion of one or more hydrophilic compounds in the haemostatic composition of the present disclosure is believed to have a beneficial effect on thrombin stability and may improve reconstitution efficiency of the dry thrombin composition. Hydrophilic compounds may also improve consistency of the haemostatic composition.

In one embodiment, the hydrophilic compound is a hydrophilic polymer. The hydrophilic polymer may be natural or synthetic, linear or branched, and have any suitable length.

In one embodiment, the hydrophilic polymer is selected from the group consisting of Polyethylenimine (PEI), Poly(ethylene glycol) (PEG), Poly(ethylene oxide), Poly(vinyl alcohol) (PVA), Poly(styrenesulfonate) (PSS), Poly(acrylic acid) (PAA), Poly(allylamine hydrochloride) and Poly(vinyl acid).

In one embodiment, the hydrophilic compound is polyethylene glycol (PEG).

In one embodiment, the hydrophilic compound is selected from the group consisting of Cetylpyridinium Chloride, Docusate Sodium, Glycine, Hypromellose, Phthalate, Lechitin, Phospholipids, Poloxamer, Polyoxyethylene Alkyl Ethers, Polyoxyethylene Castor Oil Derivatives, Polyoxyethylene Sorbitan Fatty Acid Esters, Polyoxyethylene Stearates, Polyvinyl Alcohol, Sodium Lauryl Sulfate, Sorbitan Esters (Sorbitan Fatty Acid Esters) and Tricaprylin.

In a preferred embodiment, the hydrophilic compound is a polyol. Thus, according to one embodiment of the invention, one or more polyols may be included in the haemostatic composition. Polyols may enhance the reconstitution rate of the dry thrombin composition, stabilize thrombin activity and play a role in ensuring an optimal consistency of the haemostatic composition.

A polyol as defined herein is a compound with multiple hydroxyl functional groups. Polyols include sugars (mono-, di- and polysaccharides), sugar alcohols and derivatives thereof. Especially preferred are sugar alcohols.

Monosaccharides include but are not limited to glucose, fructose, galactose, xylose and ribose.

Disaccharides include but are not limited to sucrose (saccharose), lactulose, lactose, maltose, trehalose and cellobiose.

Polysaccharides include but are not limited to starch, glycogen, cellulose and chitin.

A sugar alcohol, also known as a polyalcohol is a hydrogenated form of carbohydrate, whose carbonyl group (aldehyde or ketone, reducing sugar) has been reduced to a primary or secondary hydroxyl group (hence the alcohol). Sugar alcohols have the general formula H(HCHO)n+1H, whereas sugars have H(HCHO)nHCO. Some common sugar alcohols which may be used in the method of the present disclosure include but are not limited to: Glycol (2-carbon), Glycerol (3-carbon), Erythritol (4-carbon), Threitol (4-carbon), Arabitol (5-carbon), Xylitol (5-carbon), Ribitol (5-carbon), Mannitol (6-carbon), Sorbitol (6-carbon), Dulcitol (6-carbon), Fucitol (6-carbon), Iditol (6-carbon), Inositol (6-carbon; a cyclic sugar alcohol), volemitol (7-carbon), Isomalt (12-carbon), Maltitol (12-carbon), Lactitol (12-carbon), Polyglycitol.

In one embodiment, the haemostatic composition comprises a single hydrophilic compound, such as a single polyol.

In one embodiment of the invention, the haemostatic composition comprises more than one hydrophilic compound, such as two, three, four, five, six or even more different hydrophilic compounds.

In a preferred embodiment, the hydrophilic compound is a polyol.

In one embodiment of the invention, the haemostatic composition comprises two polyols, for example mannitol and glycerol or trehalose and a glycol.

In one embodiment of the invention, the haemostatic composition comprises one or more sugar alcohols, such as one or more sugar alcohols selected from the group consisting of Glycol, Glycerol, Erythritol, Threitol, Arabitol, Xylitol, Ribitol, Mannitol, Sorbitol, Dulcitol, Fucitol, Iditol, Inositol, volemitol, Isomalt, Maltitol, Lactitol, Polyglycitol.

In one embodiment, the haemostatic composition comprises one or more sugar alcohols and one or more sugars, such as one sugar alcohol and one sugar.

In one embodiment, the haemostatic composition comprises one sugar alcohol and optionally one or more additional hydrophilic compounds, such as one or more polyols, which may be either sugar alcohols or sugars.

In one embodiment, the haemostatic composition does not comprise a sugar as the only polyol.

In one embodiment of the invention, the haemostatic composition comprises mannitol.

In one embodiment of the invention, the haemostatic composition comprises sorbitol.

In one embodiment of the invention, the haemostatic composition comprises glycerol.

In one embodiment of the invention, the haemostatic composition comprises trehalose.

In one embodiment of the invention, the haemostatic composition comprises glycol, such as propylene glycol.

In one embodiment of the invention, the haemostatic composition comprises xylitol.

In one embodiment of the invention, the haemostatic composition comprises maltitol.

In one embodiment of the invention, the haemostatic composition comprises sorbitol.

In one embodiment, the haemostatic composition comprises from 0.01 g to 0.5 g of hydrophilic compound per gram of biocompatible polymer, such as from 0.01 g to 0.4 g, such as from 0.01 to 0.3 g, such as from 0.01 to 0.2 g, such as from 0.01 to 0.1 g, such as from 0.01 to 0.05 g of hydrophilic compound per gram of biocompatible polymer.

### Further bioactive agents

In one embodiment of the invention, the haemostatic composition comprises one or more further bioactive agents capable of stimulating haemostasis, wound healing, bone healing, tissue healing and/or tendon healing.

In one embodiment, the haemostatic composition comprises one or more further bioactive agents that stimulate bone and/or tendon and/or tissue healing such as one or more growth factors selected from the group consisting of matrix metalloproteinases (MMPs), insulin-like growth factor 1 (IGF-I), platelet-derived growth factor (PDGF), vascular endothelial growth factor (VEGF), basic fibroblast growth factor (bFGF) and transforming growth factor beta (TGF-β).

In one embodiment, the haemostatic composition comprises one or more Bone Morphogenetic Proteins (BMPs). Bone morphogenetic proteins (BMPs) are a subgroup of the TGF-β superfamily. Bone Morphogenetic Proteins (BMPs) are a group of growth factors also known as cytokines and as metabologens. Originally discovered by their ability to induce the formation of bone and cartilage, BMPs are now considered to constitute a group of pivotal morphogenetic signals, orchestrating tissue architecture throughout the body.

In one embodiment, the haemostatic composition comprises one or more matrix metalloproteinases (MMPs). MMPs are zinc-dependent endopeptidases. MMPs have a very important role in the degradation and remodeling of the extracellular matrix (ECM) during the healing process after an injury. Certain MMPs including MMP-1, MMP-2, MMP-8, MMP-13, and MMP-14 have collagenase activity, meaning that, unlike many other enzymes, they are capable of degrading collagen I fibrils.

These growth factors all have different roles during the healing process. IGF-1 increases collagen and proteoglycan production during the first stage of inflammation, and PDGF is also present during the early stages after injury and promotes the synthesis of other growth factors along with the synthesis of DNA and the proliferation of cells. The three isoforms of TGF-β (TGF-β1, TGF-β2, TGF-β3) are known to play a role in wound healing and scar formation. VEGF is well known to promote angiogenesis and to induce endothelial cell proliferation and migration.

In one embodiment, the haemostatic composition of the present disclosure comprises flakes or particles of extracelluar matrix (ECM). ECM is the extracellular part of animal tissue that usually provides structural support to the animal cells in addition to performing various other important functions. ECM has been shown to have very beneficial effect in healing as it facilitates functional tissue regeneration.

The variety of further bioactive agents that can be used in conjunction with the haemostatic composition of the invention is vast. In general, bioactive agents which may be administered via the haemostatic composition of the invention include, without limitation, antiinfectives, such as antibiotics and antiviral agents; analgesics and analgesic combinations; antihelmintics; antiarthritics; anticonvulsants; antidepressants; antihistamines; antiinflammatory agents; antimigraine preparations; antineoplastics; antiparkinsonism drugs; antipsychotics; antipyretics, antispasmodics; anticholinergics; sympathomimetics; xanthine derivatives; cardiovascular preparations including calcium channel blockers and beta-blockers such as pindolol and antiarrhythmics; antihypertensives; diuretics; vasodilators, including general coronary, peripheral and cerebral; central nervous system stimulants; hormones, such as estradiol and other steroids, including corticosteroids; immunosuppressives; muscle relaxants; parasympatholytics; psychostimulants; naturally derived or genetically engineered proteins, polysaccharides, glycoproteins, or lipoproteins; oligonucleotides, antibodies, antigens, cholinergics, chemotherapeutics, radioactive agents, osteoinductive agents, cystostatics heparin neutralizers, procoagulants and haemostatic agents, such as fibrin, fibronectin, heparinase, Factor X/Xa, Factor VII/NIIa, Factor VIII/Vllla, Factor IX/IXa, Factor XI/Xla, Factor XII/Xlla, Factor XIII/Xllla, tissue factor, batroxobin, ancrod, ecarin, von Willebrand Factor, platelet surface glycoproteins, vasopressin, vasopressin analogs, epinephrine, selectin, procoagulant venom, plasminogen activator inhibitor, platelet activating agents and synthetic peptides having haemostatic activity.

### Further compounds

The haemostatic composition of the invention may further comprise one or more of the following: DMSO (dimethyl sulfoxide) and/or 2-Methyl-2,4-pentanediol (MPD).

In one embodiment, the haemostatic composition of the present disclosure comprises one or more antimicrobial agents, such as one or more antibacterial agents.

In one embodiment, the haemostatic composition of the present disclosure comprises benzalkonium chloride (BAC).

In one embodiment, the haemostatic composition of the present disclosure does not comprise an antimicrobial agent.

In one embodiment, the haemostatic composition further comprises an extrusion enhancer, i.e. a compound which facilitates extrusion of a paste from a syringe.

It has previously been shown that the provision of certain extrusion enhancers, such as albumin in an appropriate amount, enables the use of higher gelatine concentrations as it decreases the amount of force needed to extrude the gelatine paste composition from e.g. a syringe. The use of higher gelatine concentrations may in turn improve the haemostatic properties of such products. It is necessary to provide the extrusion enhancers in appropriate amounts. The amounts are preferably high enough so as to obtain the extrusion effect, i.e. to enable a flowable paste even for relatively high amounts of the biocompatible polymer, e.g. cross-linked gelatine, so that the haemostatic composition can be accurately applied by a surgeon using e.g. a syringe comprising an applicator tip; on the other hand, the amounts shall be as low as to prevent potential negative functional properties of the haemostatic composition.

The extrusion enhancer is preferably albumin, especially human serum albumin.

In one embodiment, the haemostatic composition in paste form, i.e. after reconstitution with an aqueous medium, comprises an extrusion enhancer, such as albumin, in an amount of between about 0.1% to about 10%, such as between about 0.2% to about 8%, for example between about 0.3% to about 7%, preferably between about 0.5% to about 5%, such as between about 1% to about 4%.

In one embodiment, the haemostatic composition of the present disclosure comprises only trace amounts of albumin, such as less than 0.1%, for example less than 0.01%, such as less than 0.001%, for example less than 0.0001 %.

### Method to prepare a haemostatic composition

In one aspect, the present disclosure provides a method of preparing a haemostatic composition comprising the steps of:
a) providing the haemostatic composition as described herein, and
b) adding an amount of an aqueous medium to the haemostatic composition of a).

In one embodiment, the amount of aqueous medium added is between 2 and 12 mL per gram of haemostatic composition provided in step a), such as 2, 3, 4, 5, 6, 7, 8, 9, 10, 11 or 12 mL of aqueous medium.

In one embodiment, the amount of aqueous medium added is between 4 to 7, such as between 5 to 6 mL per gram of haemostatic composition provided in step a).

In one aspect, the present disclosure provides a method of preparing a haemostatic composition comprising the steps of:
a) providing a haemostatic composition comprising a biocompatible polymer in particulate form and fibrinogen, wherein said biocompatible polymer and fibrinogen are as described herein, and
b) adding an amount of an aqueous medium comprising thrombin.

In one embodiment, the present disclosure provides a method of preparing a haemostatic composition comprising the steps of:
a) providing a haemostatic composition comprising a biocompatible polymer in particulate form, fibrinogen and an aqueous medium, wherein said biocompatible polymer, fibrinogen and aqueous medium are as described herein, and
b) adding an amount of an aqueous medium comprising thrombin.

The components are mixed with the aqueous medium by conventional means, such as by transfer between two connected syringes, so as to form a paste.

The haemostatic composition obtained by the methods described herein is suitable for use in haemostasis and/or wound healing.

In one aspect, the present disclosure provides the haemostatic composition obtained by the methods described herein. The haemostatic composition obtained by the methods described herein is preferably a flowable paste composition.

Surprisingly, the present inventors show that the haemostatic paste compositions comprising a biocompatible polymer, thrombin and fibrinogen remain flowable after reconstitution, i.e. after mixing with an aqueous medium, for more than 1.5 hours as shown in the examples. This finding was highly unexpected since current sealants comprising thrombin and fibrinogen readily react upon combination and form a fibrin clot that will clog the device and can therefore not be deployed from a syringe for an extended period of time after combination of the components.

In one embodiment, the haemostatic composition remains flowable after addition of an aqueous medium for at least 4 hours, such as at least 2 hours, such as at least 90 minutes, such as at least 60 minutes, such as at least 30 minutes.

In one embodiment, the haemostatic composition is deployable from a syringe after addition of an aqueous medium for at least 4 hours, such as at least 2 hours, such as at least 90 minutes, such as at least 60 minutes, such as at least 30 minutes.

In one embodiment, the haemostatic composition is a paste. Thus, in one embodiment the amount of aqueous medium added to the biocompatible polymer in particulate form, such as cross-linked gelatine particles, is an amount suitable to form a paste.

In one embodiment, the haemostatic composition has a consistency within the range of about 100 g x sec to about 15,000 g x sec, such as from about 500 g x sec to about 8000 g x sec, for example from about 1000 g x sec to about 5000 g x sec, such as from about 1500 g x sec to about 3000 g x sec.

In one embodiment, the haemostatic composition has a consistency of less than about 5000 g x sec, for example less than about 4000 g x sec, such as less than about 3000 g x sec, for example less than about 2000 g x sec.

The consistency can be measured using a texture analyser (TA.XT.plus, Stable micro systems) with the following TA settings:

| Test mode | Compression |
|---|---|
| Pre-test speed | 5.00 mm/sec |
| Test speed | 0.5 mm/sec |
| Post-test speed | 10 mm/sec |
| Distance | 30.0 mm |
| Trigger type | Auto |
| Trigger force | 4.0 g |
| Probe | P/0.5R %₂" Dia Cylinder |

For a medical paste to be discharged from a syringe and an applicator tube, it should be flowable, when subjected to a force applicable for a syringe. Thus, by the term "flowable paste" is meant a paste having a viscosity facilitating a steady flow, when subjected to a force applicable for a syringe Flowability of a paste can e.g. be measured at 25-30°C and a relative humidity between 65-75%.

In one embodiment of the present disclosure, the haemostatic composition in paste form has a viscosity within the range of about 500 Pa·s to about 8000 Pa·s, such as from about 500 to about 7000 Pa·s, such as from about 500 Pa·s to about 6000 Pa·s, such as from about 600 Pa·s to about 5000 Pa·s, such as from about 700 Pa·s to about 4000 Pa·s, such as from 800 Pa·s to about 3000 Pa·s, such as from about 1000 Pa·s to about 2500 Pa·s, such as about 1500 Pa·s. In one embodiment, the haemostatic composition has a viscosity of less than about 2500 Pa·s.

The viscosity of the paste may be measured by rheometers, and preferably rotational shear based rheometers. The viscosity can be measured using a Discovery Hybrid Rheometer (DHR-1) from Waters TA instruments with controlled stress and the following measurement conditions: oscillation measurement mode with time sweep, oscillation strain of 1 %, angular frequency 1 rad/s, a 20 mm plate as upper geometry diameter, and a gap size of 1.25 mm. The measurements can be carried out at temperatures at or between 25-30 °C, and preferably at 25 °C and at a relative humidity between 65-75%.

Thus, in one embodiment not claimed, the present disclosure provides a haemostatic composition comprising:
a) one or more biocompatible polymers in particulate form,
b) fibrinogen,
c) thrombin, and
d) an aqueous medium.

Preferably, the haemostatic composition comprises an amount of aqueous medium sufficient to form a flowable paste.

In one embodiment, the aqueous medium is selected from the group consisting of water, saline, a calcium chloride solution and a buffered aqueous medium. The water may be WFI (Water For Injection). In one embodiment the aqueous medium is selected so that the reconstituted paste product is essentially isotonic. The aqueous medium is preferably sterile.

In one embodiment, the aqueous medium comprises calcium ions.

The aqueous medium of the present disclosure is in one embodiment a saline solution. In one embodiment, the aqueous medium is a calcium chloride solution.

In other embodiments, the aqueous medium is water.

The aqueous medium may also be a buffered aqueous medium. Any suitable buffering agent known to a person of skill may be used, such as one or more buffering agents selected from the group consisting of: Sodium citrate; Citric acid,; Acetic acid, Sodium acetate; K₂HPO₄, KH₂PO₄; Na₂HPO₄, NaH₂PO₄; CHES; Borax, Sodium hydroxide; TAPS; Bicine; Tris; Tricine; TAPSO; HEPES; TES; MOPS; PIPES; Cacodylate; SSC; MES, or others. The pH of the buffered aqueous medium should be suitable for creating a haemostatic composition intended for human use and can be determined by the skilled person.

The amount of aqueous medium is an amount sufficient to provide a composition in paste form, such as between 2 and 12 mL per gram of biocompatible polymer, such as from 4 to 10 mIL such as from 4 to 8 mL, such as 4 to 7 mL, such as from 5 to 6 mL of aqueous medium per gram of biocompatible polymer.

In one embodiment, the present disclosure provides a haemostatic composition comprising:
a) a biocompatible polymer in particulate form in an amount of 7 to 34% by weight, weight, wherein said biocompatible polymer is selected from gelatine, collagen, chitin, chitosan, alginate, cellulose, oxidised cellulose, carboxymethylcellulose, polyglycolic acid, polyacetic acid and combinations thereof,
b) fibrinogen in an amount of 0.008 to 5% by weight,
c) thrombin in an amount of 7 to 1700 IU per gram, and
d) an aqueous medium.

In one embodiment, the haemostatic composition comprises the biocompatible polymer in particulate form in an amount of about 7% to 20%, such as about 9% to 19%, for example about 11% to 18%, such as about 12%-17%, for example about 14%-17% by weight.

In one embodiment, the haemostatic composition comprises the biocompatible polymer in particulate form in an amount of about 7% to 20%, such as about 10% to 20%, for example about 11% to 20%, such as about 12% to 20%, for example about 13% to 20%, such as about 14% to 20%, for example about 14% to 19%, such as about 14% to 18%, for example about 14% to 17% by weight.

In one embodiment, the haemostatic composition comprises the biocompatible polymer in particulate form in an amount of about 13 to 15%, such as about 14%. In another embodiment, the haemostatic composition comprises the biocompatible polymer in particulate form in an amount of about 16 to 18%, such as about 17% by weight.

In one embodiment, the haemostatic composition comprises between about 60% to about 93% of water, for example about 70% to about 90% of water, such as between about 75% to about 90% of water, for example between about 80% to about 90% of water.

In one embodiment, the haemostatic composition, in flowable paste form, comprises fibrinogen in an amount of 0.01% to 2.5% by weight, such as 0.1% to 2% by weight, such as 0.2% to 2% by weight, such as 0.3% to 2% by weight, such as 0.4% to 2% by weight, , such as 0.4% to 1.8% by weight, such as 0.4% to 1.7% by weight, such as 0.4% to 1.6% by weight, such as 0.4% to 1.5% by weight. In one embodiment, the haemostatic composition comprises fibrinogen in an amount between 0.3 to 1.2%, such as about 0.4%, 0.5%, 0.6%, 0.7%, 0.8%, 0.9%, 1.0% or about 1.1% by weight. In one embodiment, the haemostatic composition comprises fibrinogen in an amount of 0.3 to 1.2% by weight.

In one embodiment, the haemostatic composition, in flowable paste form, comprises thrombin in an amount of 16 to 900 IU/g, such as 100 to 700 IU/g, such as 100 to 200 IU/g, such as 100 to 200 IU/g, such as 200 to 300 IU/g, such as 300 to 400 IU/g, such as 400 to 500 IU/g, such as 500 to 600 IU/g or 600 to 700 IU/g. In one embodiment, the haemostatic composition comprises thrombin in an amount of 280 to 350 IU/g of the haemostatic composition.

In one embodiment, the haemostatic composition as described herein is sterile.

### Containers

In one embodiment the haemostatic composition as described herein is provided in a first container and an aqueous solution is provided in a second container, which upon connection can form the final haemostatic paste composition to be applied to a bleeding.

The first and the second containers may be made from any suitable material such as plastic, glass, ceramic, plastic or metal, such as stainless steel. Examples of suitable plastic materials include but are not limited to polyethylene, polypropylene, polystyrene, polyvinyl chloride, and polytetrafluoroethylene (PTFE).

In one embodiment, the haemostatic composition is provided in a container which may be selected from a syringe, a vial, a jar, a tube, a tray, or a cartridge.

In a preferred embodiment said first container holding the haemostatic composition is a medical delivery device suitable for dispensing flowable haemostatic compositions to a patient in need thereof, such as a syringe, such as a single use plastic syringe.

The first container is usually made from a material suitable for chemical surface sterilisation without influencing the content of the container. For example, said first container may be made from a material which is impermeable to ethylene oxide, such as for example made from metal, glass or a plastic which is impermeable to ethylene oxide.

In one embodiment, an aqueous solution is provided in a second container which may be selected from a syringe, a vial, a jar, a tube, a tray, or a cartridge.

In a preferred embodiment the container holding the aqueous solution is a medical delivery device suitable for dispensing flowable haemostatic compositions to a patient in need thereof, such as a syringe. In one embodiment, the second container is a single-use plastic syringe.

In one embodiment, the first and second containers are interconnectable. The connector portion may be a connector portion of a standard type, such as a Luer lock or Luer slip connector. The connector portion may be provided with a threaded portion for secure connection with matching connector. The dimensions of said Luer lock or Luer slip connection may be able to change the ability of mixing in air into the haemostatic composition during mixing of the haemostatic composition and the aqueous medium. Further, the dimensions of the Luer lock or Luer slip connection may be able to influence the consistency of the haemostatic composition.

In one embodiment, the connector portion comprises a static mixer. The dimensions of said static mixer may be able to change the ability of mixing in air into the haemostatic composition during mixing of the haemostatic composition and the aqueous medium. Further, the dimensions of the static mixer may be able to influence the consistency of the haemostatic composition.

### Sterilisation

The haemostatic compositions according to the present disclosure are preferably sterile.

Thus, in one embodiment, the haemostatic composition described herein is a sterile haemostatic composition.

Any suitable sterilisation technique known in the art may be utilised. Sterilisation refers to any process that effectively kills or eliminates transmissible agents (such as fungi, bacteria, viruses, prions and spore forms etc.). Sterilisation can be achieved through e.g. application of heat, chemicals, and/or irradiation.

Sterilisation may be achieved by heat sterilisation, include autoclaving (uses steam at high temperatures) and dry heat.

Sterilisation may also be achieved by irradiation, e.g. ionizing irradiation, so as to provide sterility to the components. Such irradiation may include e-beam (beta irradiation), X-rays, gamma and beta rays, UV light and subatomic particles. The level of irradiation and conditions for sterilisation, including the time, are those that provide sterile compositions. Sterilisation conditions are similar to those currently utilized in the art and can be determined by the skilled person.

Sterilisation may be performed by chemical sterilisation such as by using ethylene oxide gas, ozone, nitrogen dioxide, chlorine bleach, glutaraldehyde, formaldehyde, ortho phthalaldehyde, hydrogen peroxide and/or peracetic acid.

The haemostatic composition or the biocompatible polymer in particulate form may also be prepared using aseptic methods.

### Medical use

The present disclosure further relates to the haemostatic composition as described herein or obtained by the methods of this disclosure for use in promoting haemostasis and/or wound, bone, tendon and/or tissue healing in an individual in need thereof.

In one embodiment, the present disclosure provides a haemostatic composition as described herein for use in promoting haemostasis in an individual in need thereof. In one embodiment, the present disclosure provides a haemostatic composition as described herein for use in promoting wound healing in an individual in need thereof.

The haemostatic composition of the present disclosure may e.g. be used in an array of surgical procedures wherein bleeding control is desired. The haemostatic composition is preferably applied to a patient in the form of a paste which conforms to irregular surfaces and is therefore useful for providing rapid haemostasis on rough or uneven surfaces where haemostatic sponges are not efficient.

Due to its superior haemostatic and adhesive properties, the haemostatic composition as disclosed herein is particularly suitable for minimally invasive/robotic surgery, where manual compression is impractical or impossible and/or where classical haemostatic fibrin sealants are ineffective. For instance, the haemostatic composition in paste form as disclosed herein can be sprayed onto a bleeding surface during minimally invasive surgery to provide a patch-like haemostatic composition which adheres sufficiently to the bleed and provides effective haemostasis without compression.

The haemostatic composition as disclosed herein has also been shown to be capable of controlling severe bleedings. Thus, in one embodiment, the haemostatic composition as disclosed herein is useful in the treatment of severe bleedings classified as level 4 or level 5 bleedings.

In general, haemostatic pastes are prepared directly at the surgical site at the time of need by the medical practitioner by addition of liquid to a container, such as a syringe, containing an amount of a haemostatic product. The haemostatic product may be pre-wetted with the liquid or be essentially dry (e.g. a free-flowing powder). The paste is thus often prepared under extremely stressful conditions and it is therefore essential that the process for preparing the paste is simple and fast to ensure that the bleeding is arrested as quickly as possible and that no mistakes are made while preparing the paste such that the nurse can keep focus on the needs of the surgeon instead of on preparing the haemostat. It is also important that the consistency of the paste is suitable for use as a haemostatic paste and that the consistency of the product is independent from preparation to preparation and over time. Currently available flowable paste products (Floseal^{®} and Surgiflo^{®}) require separate reconstitution of thrombin prior to mechanical mixing of said reconstituted thrombin solution with the biocompatible polymer by passing the biocompatible polymer and the liquid between two connected syringes a number of times to obtain a substantially homogenous paste. The reconstitution of the thrombin is time-consuming and error prone, two undesired factors in an OR setting. These products are often pre-prepared in the OR before surgery in case they are needed under surgery and unused product is often discarded causing unnecessary high OR costs.

Since the haemostatic composition of the present disclosure, in the dry, storage stable form, already includes thrombin and fibrinogen, the haemostatic paste for application to a patient can be prepared more easily since separate reconstitution and addition of e.g. thrombin is not necessary. To achieve the haemostatic composition of the present disclosure in the paste form one can simply add a suitable amount of an aqueous medium to a container comprising the haemostatic composition (in dry form) and mix by transferring the contents between two interconnected syringes a number of times, whereupon a ready-to-use haemostatic paste is formed.

A notable advantage of the compositions and methods of the present invention is that they allow for a better control of the consistency of the haemostatic compositions, while providing a superior haemostatic effect. This is highly valuable in the OR, where haemostasis needs to be achieved efficiently and as fast as possible.

Another advantage of the composition and methods of the present disclosure is that a kit consisting of fewer components can be prepared as compared to current haemostatic flowable kits. All that is required to prepare a flowable paste composition in the OR is the dry, storage-stable haemostatic composition comprised within a first container, such as a syringe, and a second container, such as a syringe, comprising an aqueous solution. Upon connection of the two and mixing, a ready-to-use flowable paste containing all necessary agents for highly effective haemostasis is formed. Thus, no extra syringes, vial adapters, needles and mixing bowls are required. This means that the manufacturing costs can be decreased and also ensures good patient safety, since there are less components for the OR staff to keep track of during surgery. Needle-free preparation of the haemostat also ensures the safety of the OR staff.

In one embodiment the present disclosure relates to a method for arresting bleeding/promoting haemostasis in an individual in need thereof by application of the haemostatic composition as disclosed herein to a site of bleeding. Upon application, the haemostatic composition is preferably in flowable, paste form.

The haemostatic composition of the present disclosure may be used for any type of surgery including general surgery, cardiothoracic surgery, vascular surgery, plastic surgery, paediatric surgery, colorectal surgery, transplant surgery, surgical oncology, trauma surgery, endocrine surgery, breast surgery, skin surgery, otolaryngology, gynaecology, oral and maxillofacial surgery, dental Surgery, orthopaedic surgery, neurosurgery, ophthalmology, podiatric surgery, urology. The haemostatic composition is particularly suitable for minimally invasive/robotic surgery where manual compression is impractical or impossible.

In one embodiment, the present disclosure relates to a method for promoting wound healing in an individual in need thereof by application of the haemostatic composition as disclosed herein to the wound.

A "wound" refers broadly to injuries to the skin and/or underlying (subcutaneous) tissue initiated in different ways (e.g., pressure sores from extended bed rest and wounds induced by trauma) and with varying characteristics. Wounds may be classified into one of four grades depending on the depth of the wound: i) Grade I: wounds limited to the epithelium; ii) Grade II: wounds extending into the dermis; iii) Grade III: wounds extending into the subcutaneous tissue; and iv) Grade IV (or full-thickness wounds): wounds wherein bones are exposed (e.g., a bony pressure point such as the greater trochanter or the sacrum). The present disclosure relates to treatment of any type of wound mentioned above using the haemostatic composition of the present disclosure.

The treatment of a wound can in principle result in healing of the wound or in accelerated healing of the wound. The accelerated healing can be a result of e.g. administration of a wound-healing promoting substance. Alternatively, the wound healing can be promoted by preventing bacterial or viral infection, or by reducing the risk of such an infection which would otherwise have prolonged the wound treatment process.

In one embodiment the present disclosure relates to a method for promoting bone and/or tendon healing in an individual in need thereof by application of the haemostatic composition as disclosed herein to the injured bone/tendon.

The "individual" referred to herein may be any mammal, including, but not limited to, mammals of the order Rodentia, such as mice and hamsters, and mammals of the order Logomorpha, such as rabbits. It is preferred that the mammals are from the order Carnivora, including Felines (cats) and Canines (dogs). It is more preferred that the mammals are from the order Artiodactyla, including Bovines (cows) and Swines (pigs) or of the order Perssodactyla, including Equines (horses). It is most preferred that the mammals are of the order Primates, Ceboids, or Simoids (monkeys) or of the order Anthropoids (humans and apes). An especially preferred mammal is the human.

In one embodiment the present disclosure relates to a haemostatic composition as disclosed herein, for use in the treatment of a wound, e.g. for arresting bleeding or for promoting wound healing.

### A haemostatic kit

The present disclosure further relates to a haemostatic kit comprising the dry haemostatic composition as described herein and an aqueous solution or an aqueous medium so that upon mixing, a haemostatic composition suitable for use in haemostasis will form.

Hence, in one embodiment the present disclosure relates to a haemostatic kit comprising:
a) a first syringe comprising a dry haemostatic composition as described herein,
b) a second syringe comprising an aqueous medium; and
c) optionally an outer package,
   wherein the two syringes are interconnectable.

Hence, in one embodiment the present disclosure relates to a haemostatic kit comprising:
a) a first syringe comprising a dry haemostatic composition comprising one or more biocompatible polymers in particulate form, such as cross-linked gelatin particles, thrombin and fibrinogen as described herein,
b) a second syringe comprising an aqueous medium; and
c) optionally an outer package,
   wherein the two syringes are interconnectable.

In one embodiment, the kit further comprises one or more applicator tips.

The kit may optionally contain instructions for use of the kit.

The components of the haemostatic kit may be as described elsewhere herein.

In one embodiment the kit comprises an outer package. The outer package is usually made from a flexible, semi-rigid or rigid material and typically consists of materials such as plastic, aluminium foil and/or plastic laminate, where the plastic may be selected from the group consisting of PET, PETG, PE, LLDPE, CPP, PA, PETP, METPET, Tyvek and optionally bonded with an adhesive, such as polyurethane, or co-extruded.

In one embodiment, the outer package is an aluminium foil outer package.

The outer package preferably forms a complete barrier to moisture.

The outer package is preferably able to endure sterilisation treatment such as by radiation.

### Examples

### Example 1: Deployability of paste compositions comprising thrombin, fibrinogen and a biocompatible polymer

### Aim

To study the time that the compositions according to the present disclosure remain deployable from a syringe.

### Materials

5 mL of a gelatine paste containing 1 g of crosslinked gelatine particles and human recombinant fibrinogen in a 10 mL syringe (first syringe). Three different amounts of fibrinogen were tested: 7, 35 and 70 mg.

1 mL of a 2000 IU of human recombinant thrombin solution in a 10 mL syringe (second syringe).

The gelatine particles used in the paste were obtained from milled crosslinked gelatine sponges (Spongostan).

### Method

The two syringes were interconnected via a luer lock and the paste was transferred back and forth between the first and the second syringes until mixed.

The mixed fibrinogen-containing pastes contained about 14% w/w of gelatine, about 285 IU of thrombin per gram of paste and about 0.1% w/w (7 mg), 0.5% w/w (35 mg) or 1% w/w (70mg) of fibrinogen, respectively.

The syringes were left at room temperature for the time periods indicated in Table 1 and the syringes were evaluated for clogging of the paste within the syringe and the ability to deploy the paste from the syringes via manual pressure on the plunger.

### Results

As can be seen in table 1, the compositions according to the present disclosure with varying amounts of fibrinogen remain deployable from a syringe for at least 90 minutes after mixing. In contrast, the compositions without biocompatible polymer clogged in the syringe and could not be deployed at any time points. Pastes containing gelatine, thrombin and 105mg fibrinogen/g gelatin were also tested (data not shown). The pastes containing 105mg fibrinogen could be deployed from the syringe but it was found that the force required to deploy the samples was markedly increased compared to the samples containing less fibrinogen and produced pastes which were more likely to break or develop cracks hence impacting paste integrity and performance.

**Table 1: Testing ability to deploy from syringe in different compositions.**

| | | **Fibrinogen (mg/g gelatin)** | | |
|---|---|---|---|---|
| **Time** | **Control^{a}** | **70** | **35** | **7** |
| 10 sec | Clogged | Pass | Pass | Pass |
| 1 min | Clogged | Pass | Pass | Pass |
| 5 min | Clogged | Pass | Pass | Pass |
| 10 min | Clogged | Pass | Pass | Pass |
| 30 min | Clogged | Pass | Pass | Pass |
| 90 min | Clogged | Pass | Pass | pass |

| | | | | |
|---|---|---|---|---|
| *^{a}Control contained no gelatin and 35 mg fibrinogen.* | | | | |

### Conclusion

The results surprisingly demonstrate that paste compositions according to the present disclosure remain deployable from a syringe for at least 90 minutes after mixing. Accordingly, paste compositions comprising a biocompatible polymer, e.g. gelatine, thrombin and fibrinogen can be prepared and remain in deployable paste form for a time period compatible with clinical use during surgery.

### Example 2: Efficacy test of gelatine-thrombin pastes comprising various amounts of fibrinogen

### Aim

To study the haemostatic efficacy of the paste compositions of Example 1, i.e. of paste compositions comprising crosslinked gelatine, thrombin and fibrinogen compared to paste compositions comprising gelatine and thrombin only. Haemostatic efficacy was tested in a porcine spleen biopsy model as described below.

### Materials and Methods

**Experimental model:** A porcine spleen biopsy-punch model was used applying 8 mm punctures (3 mm deep) in the spleen with an initial compression period of 5 seconds followed by an evaluation period of 120 seconds and following compression periods of 5 seconds.

The porcine spleen biopsy-punch model is an established model for evaluating haemostatic efficacy of haemostatic pastes *in vivo* (Hutchinson et al., 2015, Surgical Technology International XXVII). The porcine spleen biopsy-punch model of the present study is similar to the one used in Hutchinson et al., 2015.

**Experimental animal:** The pig is the animal of choice for this model since it has a large volume of blood (70 ml/kg) and a large vascular spleen that enables many haemostatic comparisons in a single animal.

**Sample preparation:** The pastes were prepared as described in example 1. Three different amounts of fibrinogen of 7, 35 and 70 mg were studied. As a control, a paste without the addition of fibrinogen was used. The chemical content and water content in the tested samples and the control were identical but for the difference in fibrinogen content.

**Surgical procedures:** A midline abdominal incision was made to expose the spleen. An 8 mm punch (3 mm deep) was made in the spleen. The bleeding intensity was evaluated on a scale from 0-5 as described herein below. Only bleeding intensities 3 and 4 were regarded as acceptable. The punch was now ready for either a control sample or a test sample. A new punch was made for every test sample. Each sample type was tested 7 times (n=7). The samples were tested in a randomized order.

A 12 minute negative control, using only wetted gauze, was performed at the initiation and completion of the testing period on each pig. The negative controls were used as an indication of the animal's ability to bleed throughout the study.

The primary test parameter was to measure the time to haemostasis (TTH). TTH is defined as the total time minus the final haemostasis evaluation period ensuring that no further bleeding occurred, i.e. no re-bleed.

The evaluation of bleeding intensity and the application of test samples and negative controls are described in detail below.

**Bleeding intensity:** The bleeding intensity of each punch was evaluated by the surgeon on a scale from 0-5 (see Table below). Bleeding intensity was noted at t=0 for each punch. Only tests performed on wounds with bleeding intensity of 3-4 were used for further analysis.

**Table: Bleeding intensity levels**

| | |
|---|---|
| Level 0 | No bleeding (for at least 30 seconds) |
| Level 1 | No bleeding observed initially, bleeding observed within the first 30 seconds of injury |
| Level 2 | Bleeding observed immediately following injury, wound site fills in approximately 30 seconds |
| Level 3 | Bleeding observed immediately following injury, wound site fills in approximately 3 seconds |
| Level 4 | Bleeding observed immediately following injury, wound site fills immediately following injury (does not include arterial or pulsating bleeding) |
| Level 5 | Bleeding observed immediately following injury, wound site fills immediately following injury (including arterial or pulsating bleeding) |

**Negative control:** Wetted gauze was placed directly on the punch. Digital pressure was applied for 30 seconds followed by a 120 second haemostasis evaluation period. Haemostasis was evaluated (defined as no sieving of blood from under test article for 30 seconds). If haemostasis was not achieved within the 120 seconds, additional 30 seconds digital pressure was applied and a 120 second re-evaluation for haemostasis was performed. Tamponade application and observation periods were performed until bleeding stopped, and haemostasis achieved, or until the testing period reached 12 minutes. Haemostasis was not achieved within the 12 minutes testing period for the negative controls, thus showing the ability of the pig to bleed throughout the study.

**Application of test samples:** Approximately 1-2 mL paste was applied directly into the punch with an applicator tip. During application the tip penetrated into the punch to ensure tissue contact. After application, gauze wetted in 0.9 % saline was placed on the punch. Digital pressure (tamponade) was applied for 5 seconds. The pressure was stopped and the gauze removed followed by evaluation of haemostasis. If no sieving of blood was seen from under the test article for 120 seconds, it was concluded that haemostasis was achieved and the experiment is ended. If blood sieved from under the test article in the 120 second time frame, the time for sieving was recorded and digital pressure was again applied for 5 seconds, after which haemostasis was inspected. This procedure was continued until haemostasis was achieved or for 12 minutes, whichever came first.

**Calculation example for evaluation of Time to Haemostasis:** 5 seconds of digital pressure, inspect for haemostasis: blood sieves after 39 seconds, digital pressure for another 5 seconds, inspect for haemostasis for 120 seconds: no sieving - conclusion: haemostasis was achieved after 5+39+5 seconds = 49 sec. i.e. the last observation period is not included in calculating the TTH.

### Results

Results are shown in Table 2. The results show that using samples containing 70 and 35 mg fibrinogen a shorter average time to haemostasis was achieved compared to 7 mg fibrinogen or control (gelatine paste with thrombin but without fibrinogen).

**Table 2: TTH of gelatine-thrombin paste composition +/- fibrinogen**

| | **Fibrinogen (mg/g gelatin)** | | | |
|---|---|---|---|---|
| | **0 (control)** | **70** | **35** | **7** |
| Time to Haemostasis^{a} / sec | 21 ± 23 | 9±10 | 9 ± 11 | 31 ± 22 |

| | | | | |
|---|---|---|---|---|
| *^{a}Values reported* as *average ± standard deviation. Each sample was tested 7 times (n=7) in randomized order.* | | | | |

### Conclusion

The paste compositions comprising gelatine, thrombin and fibrinogen led to haemostasis faster and more consistently than the control paste without fibrinogen. Thus, the present study shows that it is beneficial to include fibrinogen in haemostatic paste compositions comprising gelatine and thrombin.

### Example 3: Efficacy test of gelatine-thrombin pastes comprising fibrinogen

### Aim

To study the haemostatic efficacy of the compositions according to the present disclosure.

### Materials

1 g dry crosslinked gelatine particles, 2000 IU thrombin and 70 or 105 mg fibrinogen in a 10 mL syringe (first syringe).

6 mL of aqueous solution in a 10 mL syringe (second syringe).

The gelatine particles were obtained from milled crosslinked gelatine sponges (Spongostan) and the chemical content and water content in the tested samples and the control were identical but for the difference in fibrinogen content.

### Methods

The two syringes were interconnected via a luer lock and the 6 mL of aqueous solution was transferred to the syringe containing the dry powder composition. The resulting mixture was transferred back and forth between the first and the second syringes until mixed, resulting in a haemostatic composition contained in the second syringe.

The same porcine spleen biopsy-punch model as described in example 2 was used. Each sample type was tested 11 times (n=11). The samples were tested in a randomized order.

### Results

Results for the reconstituted haemostatic paste are shown in Table 3. The results show a shorter average time to haemostasis achieved using the reconstituted paste with fibrinogen compared to control.

**Table 3: TTH of gelatine-thrombin paste composition +/- fibrinogen**

| | **Fibrinogen (mg/g gelatin)** | | |
|---|---|---|---|
| | **0 (control)** | **70** | **105** |
| Time to Haemostasis^{a} / sec | 34 ± 28 | 15 ± 16 | 27 ± 24 |

| | | | |
|---|---|---|---|
| *^{a}Values reported as average ± standard deviation. Each sample was tested 11 times (n=11) in randomized order.* | | | |

The force required to deploy the samples with 105 mg fibrinogen was markedly increased compared to the samples containing 70 mg fibrinogen and produced pastes which were more likely to break or develop cracks hence impacting paste integrity and performance.

### Conclusion

The haemostatic compositions according to the present invention led to haemostasis faster and more consistently than the control paste without fibrinogen.

## Claims

1. A haemostatic composition comprising:
a) a biocompatible polymer in particulate form selected from gelatine, collagen, chitin, chitosan, alginate, cellulose, oxidised cellulose, carboxymethylcellulose, polyglycolic acid, polyacetic acid and combinations thereof,
b) fibrinogen in an amount of 1 to 150 mg per gram of the biocompatible polymer, and
c) thrombin in an amount of 100 to 5000 IU per gram of the biocompatible polymer.

2. The haemostatic composition according to claim 1, wherein the biocompatible polymer in particulate form is present in an amount between 80% to 99% by weight of the composition, such as between 81% to 99%, such as between 82% to 99%, such as between 83% to 99%, such as between 84% to 99%, such as between 85% to 99%, such as between 86% to 99%, such as between 87% to 99%, such as between 88% to 99%, such as between 89% to 99%, such as between 90% to 99% by weight of the composition.

3. The haemostatic composition according to any one of the preceding claims, wherein the biocompatible polymer comprises or consists of powder particles, which are substantially insoluble in an aqueous medium, such as wherein the biocompatible polymer is cross-linked, such as wherein the biocompatible polymer in particulate form comprises or consists of cross-linked gelatine particles, such as wherein the gelatine is obtained from a micronized gelatine sponge or hydrogel.

4. The haemostatic composition according to any one of the preceding claims, wherein the biocompatible polymer particles have an average size of between 1 to 1000 µm, such as between 100 and 800 µm, such as between 300 and 500 µm, such as about 450 micrometres +10% as measured by laser diffractometry.

5. The haemostatic composition according to any one of the preceding claims, wherein the fibrinogen is human fibrinogen, such as wherein the fibrinogen is recombinant human fibrinogen, such as wherein the composition comprises from 5 to 100 mg of fibrinogen per gram of the biocompatible polymer, such as from 20 to 100 mg, such as from 20 to 80 mg, such as from 30 mg to 100 mg, such as 35 mg ∓ 10% mg ∓ 10% of fibrinogen per gram of the biocompatible polymer.

6. The haemostatic composition according to any one of the preceding claims, wherein the thrombin is human thrombin, such as wherein the thrombin is recombinant human thrombin, such as wherein the composition comprises from 400 to 4000 IU of thrombin per gram of the biocompatible polymer, such as from 300 to 3000 IU, such as from 500 to 2500 IU, such as 2000 IU ∓ 10% of thrombin per gram of the biocompatible polymer.

7. The haemostatic composition according to any one the preceding claims, further comprising one or more additional active ingredient(s) capable of stimulating haemostasis, wound healing, bone healing, tissue healing and/or tendon healing, such as wherein the one or more active ingredient(s) is selected from the group consisting of: Factor XIII, tranexamic acid, bone morphogenetic proteins, metalloproteinases, insulin-like growth factor 1 (IGF-I), platelet-derived growth factor (PDGF), vascular endothelial growth factor (VEGF), basic fibroblast growth factor, transforming growth factor beta (TGF-β) and flakes or particles of extracelluar matrix (ECM).

8. The haemostatic composition according to any one the preceding claims, further comprising one or more hydrophilic compound(s), such as wherein the one or more hydrophilic compound(s) is polyethylene glycol (PEG), or is one or more polyol(s) selected from sugar alcohols, sugars and/or derivatives thereof, such as wherein the one or more hydrophilic compound(s) is a sugar alcohol selected from the group consisting of: glycol, glycerol, erythritol, threitol, arabitol, xylitol, ribitol, mannitol, sorbitol, dulcitol, fucitol, iditol, inositol, volemitol, isomalt, maltitol, lactitol, polyglycitol and mixtures thereof, such as wherein the one or more hydrophilic compound(s) is present in an amount from 0.01 g to 0.5 g of hydrophilic compound per gram of biocompatible polymer, such as from 0.01 g to 0.4 g, such as from 0.01 to 0.3 g, such as from 0.01 to 0.2 g, such as from 0.01 to 0.1 g, such as from 0.01 to 0.05 g of hydrophilic compound(s) per gram of biocompatible polymer.

9. The haemostatic composition according to any one the preceding claims, further comprising one or more extrusion enhancer(s), such as albumin, preferably human serum albumin.

10. The haemostatic composition according to any one of the preceding claims, wherein said composition contains less than 10% water by weight, preferably less than 5% water by weight, preferably less than 1% water by weight.

11. The haemostatic composition according to any one of the preceding claims, wherein the composition is sterile.

12. A method of preparing a haemostatic composition comprising the steps of:
a) providing the haemostatic composition according to any one of claims 1 to 11, and
b) adding an amount of an aqueous medium to the haemostatic composition of a).

13. A haemostatic composition obtained by the method according to claim 12, such as wherein said haemostatic composition is a flowable composition.

14. The haemostatic composition according to claim 13, wherein said composition remains flowable after addition of the aqueous medium for at least 4 hours, such at least 2 hours, such as at least 1 hour, such as at least 30 min and/or wherein said composition is deployable from a syringe after addition of the aqueous medium for at least 4 hours, such at least 2 hours, such as at least 1 hour, such as at least 30 min.

15. A haemostatic composition comprising:
a) a biocompatible polymer in particulate form in an amount of 7 to 34% by weight, wherein said biocompatible polymer is selected from gelatine, collagen, chitin, chitosan, alginate, cellulose, oxidised cellulose, carboxymethylcellulose, polyglycolic acid, polyacetic acid and combinations thereof;
b) fibrinogen in an amount of 0.008 to 5% by weight,
c) thrombin in an amount of 7 to 1700 IU per gram of the haemostatic composition, and
d) an aqueous medium.

16. The haemostatic composition according to claim 15, wherein said biocompatible polymer in particulate form comprises or consists of cross-linked gelatine particles.

17. The haemostatic composition according to any one of claims 15 to 16, wherein the biocompatible polymer is present in an amount of 10% to 30%, such as 10% to 25%, such as 10% to 20%, such as about 15% by weight; and/or wherein the fibrinogen is present in an amount of 0.01% to 2.5% by weight, such as 0.1% to 1.5% by weight, such as 0.3% to 1.2% by weight, such as 0.5% to 1.2% by weight; and/or wherein the thrombin is present in an amount of 16 to 900 IU per gram, such as 150 to 700, such as 280 to 350 IU per gram of the haemostatic composition.

18. The haemostatic composition according any one of claims 15 to 17, wherein a hydrophilic compound is present in an amount from 1% to 20% by weight, such as from 1% to 15% by weight, such as from 1% to 10% by weight.

19. The haemostatic composition according to any one of claims 15 to 18, wherein the aqueous medium is selected from the group consisting of water, saline, a calcium chloride solution, and a buffered aqueous medium.

20. The haemostatic composition according to any one of claims 15 to 19, wherein the composition is sterile.

21. The haemostatic composition according to any one of claims 1 to 11, or 13 to 20, for use in promoting haemostasis and/or wound, bone, tendon and/or tissue healing in an individual in need thereof.

22. A container comprising the haemostatic composition according to any one of claims 1 to 11 or the haemostatic composition according to any one of claims 13 to 21, such as wherein the container is an applicator, such as a syringe.

23. A kit comprising,
a) a first container, such as a syringe, comprising a composition according to any one of claims 1 to 11,
b) a second container, such as a syringe, comprising an aqueous medium; and
c) optionally an outer package,
wherein the two containers are interconnectable.

## Patentansprüche

1. Hämostatische Zusammensetzung umfassend:
a) biokompatibles Polymer in partikulärer Form ausgewählt aus Gelatine, Kollagen, Chitin, Chitosan, Alginat, Zellulose, oxidierter Zellulose, Carboxymethylcellulose, Polyglykolsäure, Polyessigsäure und Kombinationen daraus,
b) Fibrinogen in einer Menge von 1 bis 150 mg pro Gramm des biokompatiblen Polymers, und
c) Thrombin in einer Menge von 100 bis 5000 IU pro Gramm des biokompatiblen Polymers.

2. Hämostatische Zusammensetzung nach Anspruch 1, wobei das biokompatible Polymer in partikulärer Form in einer Menge zwischen 80% bis 99 Gew.-% der Zusammensetzung vorliegt, wie beispielsweise zwischen 81% bis 99%, wie beispielsweise zwischen 82% bis 99%, wie beispielsweise zwischen 83% bis 99%, wie beispielsweise zwischen 84% bis 99%, wie beispielsweise zwischen 85% bis 99%, wie beispielsweise zwischen 86% bis 99%, wie beispielsweise zwischen 87% bis 99%, wie beispielsweise zwischen 88% bis 99%, wie beispielsweise zwischen 89% bis 99%, wie beispielsweise zwischen 90% bis 99 Gew.-% der Zusammensetzung.

3. Hämostatische Zusammensetzung nach einem der vorhergehenden Ansprüche, wobei das biokompatible Polymer Pulverpartikel umfasst oder aus solchen besteht, welche in einem wässrigen Medium im Wesentlichen unlöslich sind, wie beispielsweise wobei das biokompatibles Polymer vernetzt ist, wie beispielsweise wobei das biokompatibles Polymer in partikulärer Form vernetzte Gelatinepartikel umfasst oder aus solchen besteht, wie beispielsweise wobei Gelatine aus einem mikronisierten Gelatineschwamm oder Hydrogel erhalten wird.

4. Hämostatische Zusammensetzung nach einem der vorhergehenden Ansprüche, wobei Partikel des biokompatiblen Polymers eine durchschnittliche Größe zwischen 1 bis 1000 µm aufweisen, wie beispielsweise zwischen 100 und 800 µm, wie beispielsweise zwischen 300 und 500 µm, wie beispielsweise 450 Mikrometer ±10% wie mittels Laserbeugung gemessen.

5. Hämostatische Zusammensetzung nach einem der vorhergehenden Ansprüche, wobei das Fibrinogen menschliches Fibrinogen ist, wie beispielsweise wobei das Fibrinogen rekombinantes menschliches Fibrinogen ist, wie beispielsweise wobei die Zusammensetzung 5 bis 100 mg Fibrinogen pro Gramm des biokompatiblen Polymers umfasst, wie beispielsweise 20 bis 100 mg, wie beispielsweise 20 bis 80 mg, wie beispielsweise 30 mg bis 100 mg, wie beispielsweise 35 mg ± 10% oder 70 mg ± 10% Fibrinogen pro Gramm des biokompatiblen Polymers.

6. Hämostatische Zusammensetzung nach einem der vorhergehenden Ansprüche, wobei das Thrombin menschliches Thrombin ist, wie beispielsweise wobei das Thrombin rekombinantes menschliches Thrombin ist, wie beispielsweise wobei Zusammensetzung 400 bis 4000 IU Thrombin pro Gramm des biokompatiblen Polymers umfasst, wie beispielsweise 300 bis 3000 IU, wie beispielsweise 500 bis 2500 IU, wie beispielsweise 2000 IU ± 10% Thrombin pro Gramm des biokompatiblen Polymers.

7. Hämostatische Zusammensetzung nach einem der vorhergehenden Ansprüche, weiterhin umfassend einen oder mehrere zusätzliche(n) Wirkstoff(e), die in der Lage sind, Hämostase, Wundheilung, Knochenheilung, Gewebeheilung und/oder Sehnenheilung zu stimulieren, wie beispielsweise wobei ein oder mehrere Wirkstoff(e) ausgewählt sind aus der Gruppe bestehend aus: Faktor XIII, Tranexamsäure, Knochenmorphogenetische Proteine, Metalloproteinasen, insulinähnlicher Wachstumsfaktor 1 (IGF-I), plättchenabgeleiteter Wachstumsfaktor (PDGF), vaskulärer endothelialer Wachstumsfaktor (VEGF), basischer Fibroblastenwachstumsfaktor, transformierender Wachstumsfaktor beta (TGF-β) und Flocken oder Partikel von extrazellulärer Matrix (ECM).

8. Hämostatische Zusammensetzung nach einem der vorhergehenden Ansprüche, weiterhin umfassend einen oder mehrere hydrophile(n) Verbindung(en), wie beispielsweise wobei die hydrophile Verbindung(en) Polyethylenglykol (PEG) ist/sind, oder ein oder mehrere Polyol(e) ausgewählt aus Zuckeralkoholen, Zuckern und/oder Derivaten davon ist/sind, wie beispielsweise wobei die hydrophile Verbindung ein Zuckeralkohol ist, ausgewählt aus der Gruppe bestehend aus: Glykol, Glycerol, Erythritol, Threitol, Arabitol, Xylitol, Ribitol, Mannitol, Sorbitol, Dulcitol, Fucitol, Iditol, Inositol, Volemitol, Isomalt, Maltitol, Lactitol, Polyglycitol und Mischungen davon, wie beispielsweise wobei eine oder mehrere hydrophile Verbindung(en) in einer Menge von 0,01 g bis 0,5 g hydrophiler Verbindung pro Gramm biokompatibles Polymer vorhanden ist/sind, wie beispielsweise von 0,01 g bis 0,4 g, wie beispielsweise von 0,01 bis 0,3 g, wie beispielsweise von 0,01 bis 0,2 g, wie beispielsweise von 0,01 bis 0,1 g, wie beispielsweise von 0,01 bis 0,05 g hydrophiler Verbindung(en) pro Gramm biokompatibles Polymer.

9. Hämostatische Zusammensetzung nach einem der vorhergehenden Ansprüche, weiterhin umfassend einen oder mehrere Extrusionshilfstoffe, wie beispielsweise Albumin, bevorzugt humanes Serumalbumin.

10. Hämostatische Zusammensetzung nach einem der vorhergehenden Ansprüche, wobei die Zusammensetzung weniger als 10 Gew.-% Wasser enthält, bevorzugt weniger als 5 Gew.-% Wasser, bevorzugt weniger als 1 Gew.-% Wasser.

11. Hämostatische Zusammensetzung nach einem der vorhergehenden Ansprüche, wobei die Zusammensetzung steril ist.

12. Verfahren zur Herstellung einer hämostatischen Zusammensetzung umfassend die Schritte:
a) Bereitstellen der hämostatischen Zusammensetzung nach einem der Ansprüche 1 bis 11, und
b) Zugabe einer Menge eines wässrigen Mediums zu der hämostatischen Zusammensetzung von a).

13. Hämostatische Zusammensetzung erhalten durch das Verfahren nach Anspruch 12, wie beispielsweise wobei die hämostatische Zusammensetzung eine fließfähige Zusammensetzung ist.

14. Hämostatische Zusammensetzung nach Anspruch 13, wobei die Zusammensetzung nach Zugabe des wässrigen Mediums für mindestens 4 Stunden fließfähig bleibt, wie mindestens 2 Stunden, wie mindestens 1 Stunde, wie mindestens 30 min und/oder wobei die Zusammensetzung nach Zugabe des wässrigen Mediums für mindestens 4 Stunden aus einer Spritze applizierbar ist, wie mindestens 2 Stunden, wie mindestens 1 Stunde, wie mindestens 30 min.

15. Hämostatische Zusammensetzung umfassend:
a) ein biokompatibles Polymer in partikulärer Form in einer Menge von 7 bis 34 Gew.-%, wobei das biokompatible Polymer ausgewählt ist aus Gelatine, Kollagen, Chitin, Chitosan, Alginat, Zellulose, oxidierter Zellulose, Carboxymethylcellulose, Polyglykolsäure, Polyessigsäure und Kombinationen davon;
b) Fibrinogen in einer Menge von 0,008 bis 5 Gew.-%,
c) Thrombin in einer Menge von 7 bis 1700 IU pro Gramm der hämostatischen Zusammensetzung, und
d) wässriges Medium.

16. Hämostatische Zusammensetzung nach Anspruch 15, wobei das biokompatible Polymer in partikulärer Form vernetzte Gelatinepartikel umfasst oder aus solchen besteht.

17. Hämostatische Zusammensetzung nach einem der Ansprüche 15 bis 16, wobei das biokompatible Polymer in einer Menge von 10% bis 30%, wie 10% bis 25%, wie 10% bis 20%, wie etwa 15 Gew.-% vorliegt; und/oder wobei das Fibrinogen in einer Menge von 0,01% bis 2,5 Gew.-% vorliegt, wie 0,1% bis 1,5 Gew.-%, wie 0,3% bis 1,2 Gew.-%, wie 0,5% bis 1,2 Gew.-%; und/oder wobei das Thrombin in einer Menge von 16 bis 900 IU pro Gramm vorliegt, wie 150 bis 700, wie 280 bis 350 IU pro Gramm der hämostatischen Zusammensetzung.

18. Hämostatische Zusammensetzung nach einem der Ansprüche 15 bis 17, wobei die hydrophile Verbindung in einer Menge von 1% bis 20 Gew.-% vorliegt, wie von 1% bis 15 Gew.-%, wie von 1% bis 10 Gew.-%.

19. Hämostatische Zusammensetzung nach einem der Ansprüche 15 bis 18, wobei das wässrige Medium ausgewählt ist aus der Gruppe bestehend aus Wasser, Kochsalzlösung, Calciumchloridlösung und gepuffertem wässrigem Medium.

20. Hämostatische Zusammensetzung nach einem der Ansprüche 15 bis 19, wobei die Zusammensetzung steril ist.

21. Hämostatische Zusammensetzung nach einem der Ansprüche 1 bis 11 oder 13 bis 20, zur Verwendung zur Förderung von Hämostase und/oder Wund-, Knochen-, Sehnen- und/oder Gewebeheilung bei einem Individuum, das diese benötigt.

22. Behälter umfassend die hämostatische Zusammensetzung nach einem der Ansprüche 1 bis 11 oder die hämostatische Zusammensetzung nach einem der Ansprüche 13 bis 21, wie beispielsweise wobei der Behälter ein Applikator ist, wie beispielsweise eine Spritze.

23. Kit umfassend,
a) einen ersten Behälter, wie beispielsweise eine Spritze, umfassend die Zusammensetzung nach einem der Ansprüche 1 bis 11,
b) einen zweiten Behälter, wie beispielsweise eine Spritze, umfassend wässriges Medium; und
c) optional eine äußere Verpackung,
wobei die beiden Behälter miteinander verbindbar sind.

## Revendications

1. Composition hémostatique comprenant:
a) un polymère biocompatible sous forme particulaire sélectionné parmi gélatine, collagène, chitine, chitosane, alginate, cellulose, cellulose oxydée, carboxyméthylcellulose, acide polyglycolique, acide polyacétique et combinaisons de ceux-ci,
b) fibrinogène en une quantité de 1 à 150 mg par gramme de polymère biocompatible, et
c) thrombine en une quantité de 100 à 5000 UI par gramme de polymère biocompatible.

2. Composition hémostatique selon la revendication 1, dans laquelle le polymère biocompatible sous forme particulaire est présent en une quantité comprise entre 80% et 99% en poids de la composition, telle que comprise entre 81% et 99%, telle que comprise entre 82% et 99%, telle que comprise entre 83% et 99%, telle que comprise entre 84% et 99%, telle que comprise entre 85% et 99%, telle que comprise entre 86% et 99%, telle que comprise entre 87% et 99%, telle que comprise entre 88% et 99%, telle que comprise entre 89% et 99%, telle que comprise entre 90% et 99% en poids de la composition.

3. Composition hémostatique selon l'une quelconque des revendications précédentes, dans laquelle le polymère biocompatible comprend ou consiste en des particules de poudre, qui sont sensiblement insolubles dans un milieu aqueux, telle que dans laquelle le polymère biocompatible est réticulé, telle que dans laquelle le polymère biocompatible sous forme particulaire comprend ou consiste en des particules de gélatine réticulée, telle que dans laquelle la gélatine est obtenue à partir d'une éponge de gélatine micronisée ou d'un hydrogel.

4. Composition hémostatique selon l'une quelconque des revendications précédentes, dans laquelle les particules de polymère biocompatible ont une taille moyenne comprise entre 1 et 1000 µm, telle que comprise entre 100 et 800 µm, telle que comprise entre 300 et 500 µm, telle que 450 micromètres 10% telle que mesurée par diffractométrie laser.

5. Composition hémostatique selon l'une quelconque des revendications précédentes, dans laquelle le fibrinogène est un fibrinogène humain, telle que dans laquelle le fibrinogène est un fibrinogène humain recombinant, telle que dans laquelle la composition comprend de 5 à 100 mg de fibrinogène par gramme de polymère biocompatible, telle que de 20 à 100 mg, telle que de 20 à 80 mg, telle que de 30 mg à 100 mg, telle que 35 mg ± 10% ou 70 mg ± 10% de fibrinogène par gramme de polymère biocompatible.

6. Composition hémostatique selon l'une quelconque des revendications précédentes, dans laquelle la thrombine est une thrombine humaine, telle que dans laquelle la thrombine est une thrombine humaine recombinante, telle que dans laquelle la composition comprend de 400 à 4000 UI de thrombine par gramme de polymère biocompatible, telle que de 300 à 3000 UI, telle que de 500 à 2500 UI, telles que 2000 IU ± 10% de thrombine par gramme de polymère biocompatible.

7. Composition hémostatique selon l'une quelconque des revendications précédentes, comprenant en outre un ou plusieurs ingrédient(s) actif(s) supplémentaire(s) capable(s) de stimuler l'hémostase, la cicatrisation de plaie, la cicatrisation osseuse, la cicatrisation tissulaire et/ou la cicatrisation de tendon, telle que dans laquelle le ou les ingrédient(s) actif(s) supplémentaire(s) est sélectionné parmi le groupe constitué de: Facteur XIII, acide tranexamique, protéines morphogénétiques osseuses, métalloprotéinases, facteur de croissance analogue à l'insuline 1 (IGF-I), facteur de croissance dérivé des plaquettes (PDGF), facteur de croissance endothélial vasculaire (VEGF), facteur de croissance fibroblastique basique, facteur de croissance transformant bêta (TGF-ß) et flocons ou particules de matrice extracellulaire (ECM).

8. Composition hémostatique selon l'une quelconque des revendications précédentes, comprenant en outre un ou des composé(s) hydrophile(s), telle que dans laquelle le ou les composé(s) hydrophile(s) est le polyéthylène glycol (PEG), ou est un ou plusieurs polyol(s) sélectionné(s) parmi alcools de sucre, sucres et/ou dérivés de ceux-ci, telle que dans laquelle le ou les composé(s) hydrophile(s) est un alcool de sucre sélectionné parmi le groupe constitué de: glycol, glycérol, érythritol, thréitol, arabitol, xylitol, ribitol, mannitol, sorbitol, dulcitol, fucitol, iditol, inositol, volemitol, isomalt, maltitol, lactitol, polyglycitol et mélanges de ceux-ci, telle que que dans laquelle le ou les composé(s) hydrophile(s) est présent en une quantité de 0,01 g à 0,5 g de composé hydrophile par gramme de polymère biocompatible, telle que de 0,01 g à 0,4 g, telle que de 0,01 à 0,3 g, telle que de 0,01 à 0,2 g, telle que de 0,01 à 0,1 g, telle que de 0,01 à 0,05 g de composé(s) hydrophile(s) par gramme de polymère biocompatible.

9. Composition hémostatique selon l'une quelconque des revendications précédentes, comprenant en outre un ou plusieurs agent(s) facilitant l'extrusion, tel que de l'albumine, de préférence de l'albumine sérique humaine.

10. Composition hémostatique selon l'une quelconque des revendications précédentes, dans laquelle ladite composition contient moins de 10% d'eau en poids, de préférence moins de 5% d'eau en poids, de préférence moins de 1% d'eau en poids.

11. Composition hémostatique selon l'une quelconque des revendications précédentes, dans laquelle la composition est stérile.

12. Procédé de préparation d'une composition hémostatique comprenant les étapes de:
a) fournir la composition hémostatique selon l'une quelconque des revendications 1 à 11, et
b) ajouter une quantité d'un milieu aqueux à la composition hémostatique de a).

13. Composition hémostatique obtenue par le procédé selon la revendication 12, telle que dans laquelle ladite composition hémostatique est une composition fluide.

14. Composition hémostatique selon la revendication 13, dans laquelle ladite composition reste fluide après addition du milieu aqueux pendant au moins 4 heures, telle qu'au moins 2 heures, telle qu'au moins 1 heure, telle qu'au moins 30 min et/ou dans laquelle ladite composition est expulsable d'une seringue après addition du milieu aqueux pendant au moins 4 heures telle qu'au moins 2 heures, telle qu'au moins 1 heure, telle qu'au moins 30 min.

15. Composition hémostatique comprenant:
a) un polymère biocompatible sous forme particulaire en une quantité de 7 à 34% en poids, dans laquelle ledit polymère biocompatible est sélectionné parmi gélatine, collagène, chitine, chitosane, alginate, cellulose, cellulose oxydée, carboxyméthylcellulose, acide polyglycolique, acide polyacétique et combinaisons de ceux-ci;
b) fibrinogène en une quantité de 0,008 à 5% en poids,
c) thrombine en une quantité de 7 à 1700 UI par gramme de la composition hémostatique, et
d) milieu aqueux.

16. Composition hémostatique selon la revendication 15, dans laquelle ledit polymère biocompatible sous forme particulaire comprend ou consiste en des particules de gélatine réticulée.

17. Composition hémostatique selon l'une quelconque des revendications 15 à 16, dans laquelle le polymère biocompatible est présent en une quantité de 10% à 30%, telle que 10% à 25%, telle que 10% à 20%, telle que environ15% en poids; et/ou dans laquelle le fibrinogène est présent en une quantité de 0,01% à 2,5% en poids, telle que 0,1% à 1,5% en poids, telle que 0,3% à 1,2% en poids, telle que 0,5% à 1,2% en poids; et/ou dans laquelle thrombine est présente en une quantité de 16 à 900 UI par gramme, telle que 150 à 700, telle que 280 à 350 UI par gramme de la composition hémostatique.

18. Composition hémostatique selon l'une quelconque des revendications 15 à 17, dans laquelle un composé hydrophile est présent en une quantité de 1% à 20% en poids, telle que de 1% à 15% en poids, telle que de 1% à 10% en poids.

19. Composition hémostatique selon l'une quelconque des revendications 15 à 18, dans laquelle le milieu aqueux est sélectionné parmi le groupe constitué d'eau, de solution saline, de solution de chlorure de calcium, et d'un milieu aqueux tamponné.

20. Composition hémostatique selon l'une quelconque des revendications 15 à 19, dans laquelle la composition est stérile.

21. Composition hémostatique selon l'une quelconque des revendications 1 à 11, ou 13 à 20, pour utilisation dans la promotion de l'hémostase et/ou la cicatrisation de plaie, d'os, de tendon et/ou de tissu chez un individu en ayant besoin.

22. Conteneur comprenant la composition hémostatique selon l'une quelconque des revendications 1 à 11 ou la composition hémostatique selon l'une quelconque des revendications 13 à 21, tel que dans laquelle le conteneur est un applicateur, tel que une seringue.

23. Kit comprenant,
a) un premier conteneur, tel qu'une seringue, comprenant une composition selon l'une quelconque des revendications 1 à 11,
b) un second conteneur, tel qu'une seringue, comprenant un milieu aqueux; et
c) éventuellement un emballage extérieur,
dans lequel les deux conteneurs sont interconnectables.
